# EUROPEAN PATENT SPECIFICATION

(11) **EP 1 274 454 B1**
(45) Date of publication and mention of the grant of the patent: **28.12.2005**
(21) Application number: 01930632.3
(22) Date of filing: 20.04.2001
(51) Int. Cl.: A61K 39/39

(54) **METHODS AND COMPOSITIONS FOR ELICITING AN IMMUNE RESPONSE**
METHODEN UND ZUSAMMENSETZUNGEN ZUR INDUZIERUNG EINER IMMUNANTWORT
METHODES ET COMPOSITIONS D'INDUCTION D'UNE REPONSE IMMUNITAIRE

(30) Priority: 21.04.2000 US 198839 P; 12.04.2001 US 834814
(43) Date of publication of application: 15.01.2003
(73) Proprietor: Chemocentryx, Inc., San Carlos, CA 94070 (US)
(72) Inventor: SCHALL, Thomas, J., San Carlos,CA 94070 (US); TALBOT, Dale, San Francisco, CA 94127 (US)
(74) Representative: Raynor, John
(86) International application number: PCT/US2001/012950
(87) International publication number: WO 2001/080882

(56) References cited:
- WO-A-99/29728
- WO-A-99/43839
- WO-A-99/53960
- EO SEONG KUG ET AL: "Modulation of immunity against herpes simplex virus infection via mucosal genetic transfer of plasmid DNA encoding chemokines." JOURNAL OF VIROLOGY, vol. 75, no. 2, January 2001 (2001-01), pages 569-578, XP002187584 ISSN: 0022-538X

## Description

### I. Background

Immunization, or vaccination, is a widely used method to elicit an immune response to an antigen for prophylactic purposes. For example, by administering a harmless form of an antigen from a pathogen, such as an attenuated virus, the production of antibodies and stimulation of immune cells specific for the harmful form of the pathogen occurs. However, present immunization methods are not effective for all antigens. Moreover, there is a considerable lag time from immunization until the immune system provides protection for the subject. Thus, improved methods and reagents for vaccination are desired by the medical community.

WO-A_99 53960 discloses medicaments which comprising an antigen and a chemokine (as an adjuvant) to be selected from a large number of possible chemokines.

Similarly WO-A-99 29728 describes the use of chemokines and antigens for the preparation of a medicament, and WO-A-99 43839 describes the use of chemokines and antigens in the preparation of pharmaceutical compositions.

However, none of the above documents identify any specific chemokines as being particularly advantageous, in particular for attracting dendritic cells and/or macrophages, in such compositions.

### II. Summary

The present invention provides compositions useful for attracting antigen-presenting cells to a site of administration, and new vaccines and immunization methods. According to one aspect of the present invention, an antigen-presenting cell chemotaxin (APC-chemotaxin) and an antigen are used in the manufacture of a medicament, wherein the APC-chemotaxin is:
(a) a chemokine selected from murine cytomegalovirus chemokine-2 (vMCK-2) and mC10;
(b) a variant of (a) that does not change the chemotactic properties of the polypeptide; or
(c) a polynucleotide encoding (a) or (b).

According to another aspect of the present invention, a vaccine composition is provided comprising a substantially purified chemokine polypeptide and an antigenic polypeptide, wherein the chemokine polypeptide is murine cytomegalovirus chemokine-2 (vMCK-2) or a variant thereof that does not change the chemotactic properties of the polypeptide, or mC10 or a variant thereof that does not change the chemotactic properties of the polypeptide.

According to a further aspect, a composition is provided comprising a substantially purified antigen-presenting cell chemotaxin (APC-chemotaxin) and an antigen, wherein said APC-chemotaxin is:
(a) a chemokine selected from the group consisting of murine cytomegalovirus chemokine-2 (vMCK-2) or mC10;
(b) a variant of (a) that does not change the chemotactic properties of the polypeptide; or
(c) a polynucleotide encoding (a) or (b).

Optionally, the composition may further comprise a pharmacetucially acceptable excipient or conventional adjuvant.

According to a further aspect, a method is provided of formulating a composition capable of inducing an immune response to a specified antigen in a subject, comprising combining an antigen-presenting cell chemotaxin (APC-chemotaxin), the specified antigen and a pharmaceutically acceptable excipient or a conventional adjuvant, wherein the APC-chemotaxin is:
(a) a chemokine selected from murine cytomegalovirus chemokine-2 (vMCK-2) and mC10;
(b) a variant of (a) that does not change the chemotactic properties of the polypeptide; or
(c) a polynucleotide encoding (a) or (b).

In one embodiment, the medicament, vaccine or compositions of the present invention may further comprise an additional chemokine. In particular, the medicament, vaccine or composition may comprise both vMCK-2 and mC10. The APC-chemotaxin may be encoded by a polynucleotide produced by in vitro recombination of the polynucleotides that encode vMCK-2 or mC10 and another naturally occurring chemokine.

As appropriate, additional chemokines may be selected from hMIP1α, hMIP1α (70aa), mMIP-1α, hRANTES, hMET-RANTES, mRANTES, hHCC-1, hMPIF-1, hMPIF-1 (22-137), hMPIF-1 (46-137), hMIP-1δ, hMCP-4, mMCP-5, mMARC, mEotaxin, mMCP-1(JE), mTECK, mMIP-2, mBLC, hLeukotactin, mMIG, mMIP-1β. hMCP-2, hMCP-3, vMIP-1, hMIP-3α, hMIP-3β, mMDC, hMIP-1β, and/or mMIP-1γ

In one emodiment, the antigen may be derived from a microbial pathogen or a tumor-associated antigen. In particular, the microbial pathogen may be a bacterium or a virus. The APC-chemotaxinand the antigen (eg. Immunogen) may administered (e.g., by injection) at the same time, for example, by coinjection of the immunogen and the APC-chemotaxin. Alternatively, the immunogen may be administered to the subject after the APC-chemotaxin is administered.

As noted, each of the aforedescribed compositions is useful for attracting antigen-presenting cells to a specified site, e.g., a site of antigen concentration, for example into or near a solid tumor.

Equally, the compositions are useful for stimulating the innate immune response of the subject.

### III. Figures

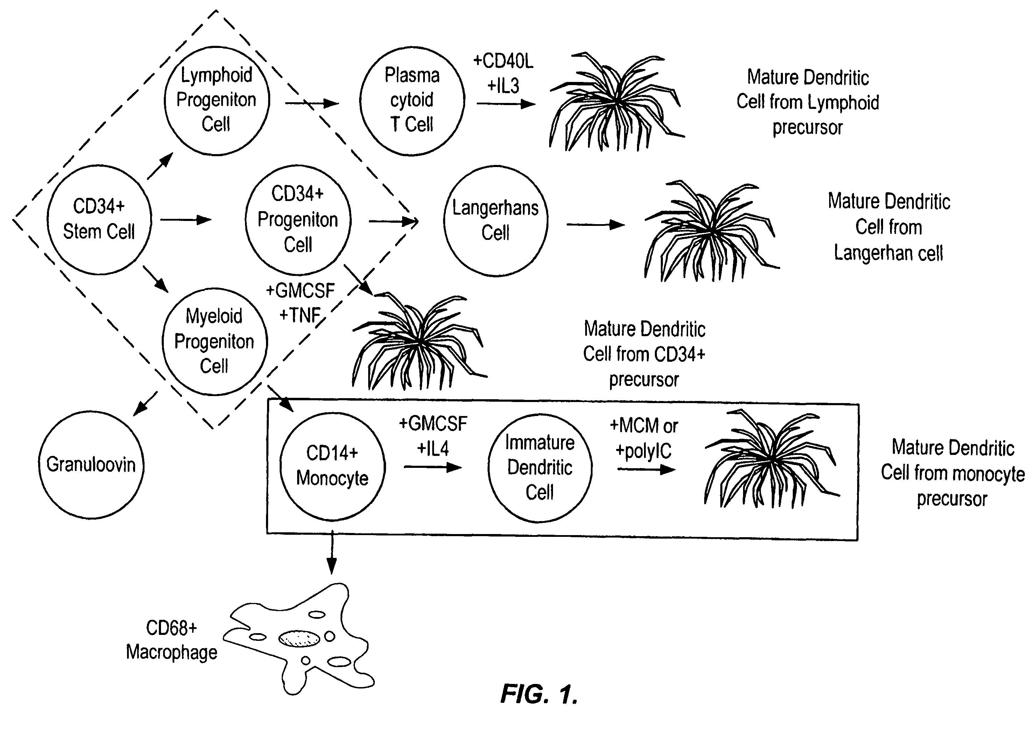

Figure 1 shows developmental pathways of dendritic cells.
Figure 2 shows the sequence of exemplary chimeric chemokines (seq.id.nos:4-7) and chemokines from which these are derived (seq.id.nos:1-3).

### IV. Definitions and Abbreviations

As used herein, a cell sample or cell population is "substantially pure" or "substantially purified" if at least about 75%, preferably at least about 85%, often at least about 90%, at least about 95% or more of the total cells in the sample are of a defined type (e.g., in a substantially pure preparation of dendritic cells, at least about, e.g., 95% of the total number of cells are dendritic cells, while in a substantially pure preparation of immature dendritic cells, at least about, e.g., 95% of the total number of cells are immature dendritic cells.

"Polynucleotide," as used herein, refers to a deoxyribonucleotide (DNA) or ribonucleotide (RNA) in either single- or double-stranded form, and encompasses known analogs of natural nucleotides which can function in a manner similar to the naturally occurring nucleotides. The phrase "polynucleotide encoding" refers to a nucleic acid sequence which directs the expression of a specific protein or peptide. The nucleic acid sequences include both the DNA sequence that is transcribed into RNA and the RNA sequence that is translated into the protein. The nucleic acid sequence includes both the full length nucleic acid sequence as well as non-full length sequences derived from the full length sequence.

As used herein, a "subject" is a mammal such as a primate (e.g., human patient or volunteer, or non-human primate), or other animal such as a rat, mouse, rodent, rabbit (e.g., experimental animals), and the like, and including agriculturally important mammals such as, without limitation, a goat, pig, cow, sheet, horse, and the like, and pets such as dogs, cats, and the like.

As used herein, the "substantially sequence identity," refers to two or more sequences or subsequences that have at least 60%, at least 70%, at least 80%, or at least 90%, 95%, 98%, or 99% nucleotide or amino acid residue identity, when compared and aligned for maximum correspondence, as measured using one of the following sequence comparison algorithms or by visual inspection. Two sequences (amino acid or nucleotide) can be compared over their full-length (e.g., the length of the shorter of the two, if they are of substantially different lengths) or over a subsequence such as at least about 50, about 100, about 200, about 500 or about 1000 contiguous nucleotides or at least about 10, about 20, about 30, about 50 or about 100 contiguous amino acid residues.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, 1981, *Adv. Appl. Math.* 2:482, by the homology alignment algorithm of Needleman & Wunsch, 1970, *J. Mol. Biol*. 48:443, by the search for similarity method of Pearson & Lipman, 1988, *Proc. Natl. Acad. Sci. USA* 85:2444, by computerized implementations of these algorithms (GAP, BESTFIT, FASTA, and TFASTA in the Wisconsin Genetics Software Package, Genetics Computer Group, 575 Science Dr., Madison, WI), or by visual inspection (see generally Ausubel et al., supra). Each of these references and algorithms is incorporated by reference herein in its entirety. When using any of the aforementioned algorithms, the default parameters for "Window" length, gap penalty, etc., are used.

One example of algorithm that is suitable for determining percent sequence identity and sequence similarity is the BLAST algorithm, which is described in Altschul et al., 1990, *J. Mol. Biol*. 215:403-410. Software for performing BLAST analyses is publicly available through the National Center for Biotechnology Information (http://www.ncbi.ntm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al, supra). These initial neighborhood word hits act as seeds for initiating searches to find longer HSPs containing them. The word hits are then extended in both directions along each sequence for as far as the cumulative alignment score can be increased. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLAST program uses as defaults a wordlength (W) of 11, the BLOSUM62 scoring matrix (see Henikoff & Henikoff, 1989, *Proc. Natl. Acad. Sci. USA* 89:10915) alignments (B) of 50, expectation (E) of 10, M=5, N=-4, and a comparison of both strands.

Abbreviations: When referring to a chemokine "h" means human and "m" means murine. In some cases, Latin letters are used instead of the Greek α, β, or y. Thus, as used herein, mMIP-1a means murine MIP-1α.

### V. Detailed Description

The invention provides compositions useful for attracting antigen-presenting cells to a site of administration. In an aspect, the present invention provides compositions and methods for inducing or enhancing an immune response to an antigen in a subject. The compositions and methods are useful for, among other things, formulation of vaccines and for therapeutic and prophylactic vaccination (immunization) protocols and for production of useful antibodies (e.g., monoclonal antibodies for therapeutic or diagnostic use).

In particular, the present invention relates to compositions that contain an agent that is chemotactic for dendritic cells (DCs) and/or macrophages (together referred to as "antigen-presenting cells" or "APCs"). For convenience, the chemotactic agent is sometimes referred to as a "APC-chemotaxin," and the composition containing the APC-chemotaxin (which may contain excipients or other components) is sometimes referred to herein as "the chemotactic composition" or "the composition."

In some embodiments of the invention, the APC-chemotaxin is specifically chemotactic for specified cell types (e.g., dendritic cells) or cell developmental stages (e.g., immature dendritic cells). In related embodiments the agent, while chemotactic for some cells, is *not* chemotactic for certain cell-types (e.g., neutrophils) or cell developmental stages (e.g., mature dendritic cells). Chemotaxis is determined using one or more of the assays described herein.

The present invention further involves administration of an antigen, in addition to the chemotactic composition. Thus, in one embodiment, the chemotactic composition and an antigen are administered to the same physical site in the subject. For example, the antigen may be combined with the chemotactic composition, and the mixture administered (e.g., injected) together. Alternatively, the composition and the antigen are administered separately to the same area of the subject (e.g., injected to the same site, applied topically to the same site, and the like). In some embodiments, as described in detail *infra,* the composition and antigen are administered at different times.

Without intending to be bound by a particular mechanism, it is believed that the APC-chemotaxin(s) promote(s) an immune reaction to the antigen by recruiting APCs to areas of antigen contact. Antigens are taken up by APCs and partially degraded. Subsequently, a fraction of the degraded antigen is presented with MHC class I or II molecules on the surface of the APC. Such cells stimulate proliferation of either cytotoxic T cells or helper T cells, or the production of antibody by B-cells.

It will be apparent that, in one aspect, the invention provides, new methods and reagents useful for therapeutic and prophylactic immunization (i.e., the deliberate provocation, enhancement or intensification, of an adaptive immune response). Particular advantages expected over prior immunization methods include: an accelerated immune response in a host following administration of antigen, a more effective response to administration of, or exposure to, very small quantities of an antigen (e.g., toxin or pathogen) due to increased antigen uptake by APCs, and more effective anti-tumor therapies (e.g., due to enhanced tumor antigen uptake by host APCs).

As used herein, an "immune response" means (unless otherwise specified) an adaptive immune response to a specific antigen. In one aspect, an immune response involves the concerted action of lymphocytes, antigen presenting cells, phagocytic cells, and various soluble macromolecules in defending the body against infection or other exposure to non-self molecules. The immune response can be detected and quantified (e.g., following immunization) by measuring cellular or humoral responses according to numerous assays known in the art (see, e.g., Coligan et al., 1991 (suppl. 1999), CURRENT PROTOCOLS IN IMMUNOLOGY, John Wiley & Sons (hereinafter, sometimes "Coligan")). For example, to detect a cellular immune response, T cell effector effects against cells expressing the antigen are detected using standard assays, e.g., target-cell killing, macrophage activation, B-cell activation or lymphokine production. Humoral responses are measured by detecting the appearance of, or increase in titer of, antigen-specific antibodies using routine methods such as ELISA. The progress of the antibody response can be determined by measuring class switching (i.e., the switch from an early IgM response to a later IgG response.

Various aspects of the invention will now be described in greater detail, to provide guidance to the practitioner.

### A. Chemotactic compositions

According to the invention, a composition containing an APC-chemotaxin is administered to induce or enhance an immune response in a subject. In various embodiments, the chemotaxin attracts dendritic cells, macrophages, or both. Chemotaxins that attract dendritic cells, particularly immature dendritic cells, are especially useful. In a particularly useful embodiment, the chemotaxin has a high level of specificity for a particular cell type, developmental stage, or both. Thus, in one embodiment, the APC-chemotaxin attracts immature dendritic cells, but does not attract one or more of the following other classes of cells: mature dendritic cells, neutrophils, monocytes, T cells, B cells, eosinophils, mast cells, red blood cells, and progenitor cells.

APC-chemotaxins may be any of a large variety of compounds, both naturally occurring and synthetic, organic and inorganic, and including polymers (e.g., oligopeptides, polypeptides, oligonucteotides, and polynucleotides), small molecules, antibodies, sugars, fatty acids, nucleotides and nucleotide analogs, analogs of naturally occurring structures (e.g., peptide mimetics, nucleic acid analogs, and the like), and numerous other compounds. In the present invention, the composition contains at least one of mC10, vMCK-2 or a variant of an aforementioned chemokine, such as a recombinant polypeptide produced by *in vitro* mutation, *in vitro* recombination or shuffling of a polynucleotide encoding the chemokines, a polynucleotide encoding at least one of the aforementioned chemokines or variant molecules, or a synthetic (i.e., chemically synthesized) polypeptide variant (e.g., mimetic) of one or more of these chemokines.

Reagents and methods for, *inter alia,* identifying APC-chemotaxins, and preparing and administering the compositions of the invention are described *infra.*

### 1. Assays for Identification of APC-Chemotaxins

In one aspect of the invention, APC-chemotaxins are identified using *in vivo* or *in vitro* assays.

### a) In vitro assays

The APC-chemotaxins used in the methods of the invention have certain properties, which can be detected in *in vitro* chemotaxis assays. *In vitro* chemotaxis assays are well known. Migration assays are often carried out by physically separating the cells from the chemoattractant using a porous membrane and allowing the cells to move directionally, up a diffusion gradient of the chemotaxin (see, e.g., Keller, 1972, *Agents Actions* 2:161-69; Gee A.P., 1984, *Mol. Cell. Biochem.* 62:5-11; Keller et al., 1974, *Antibiot. Chemother.* 19:112-25).

A variety of assay configurations are known and are suitable in the practice of the present invention. Most often, standard filter based assays are used because they are convenient, robust, and relatively inexpensive. These filter based assays include a classical or modified Boyden chamber and variants (see, e.g., Bozarth et al., 1997, *Meth. Cell Science*, 19:179-187; Frevert et al., 1998, *J. Imm. Methods,* 213:41-52; Penno et al., 1997, *Meth. Cell Science,* 19:189-195; O'Leary et al., 1997, *Am. J. Resp. Cell and Mol. Bio.,* 16:267-274; Falk, et al., 1980, *J. Imm. Methods,* 33: 239-247; Harvath, et al., 1980, *J. Imm. Methods,* 37:39-45; Richards et al., 1984, *Immunological Communications,* 13:49-62; Falk et al., 1982, *Infection and Immunity* 36:450-454; Harvath et al., 1982, *Infection and Immunity* 36:443-449). In filter-based assays, cells are placed in a compartment separated from a candidate chemotaxin by a filter through which the candidate chemotaxin can diffuse. After an incubation period, the number of cells (or percentage of total cells) that have migrated onto or through the filter is determined. Migration of cells at a level above background (i.e., migration in the absence of the candidate chemotaxin, e.g., in the presence of only a carrier such as PBS or Chemotaxis buffer *(infra)* indicates that the candidate chemotaxin is indeed chemotactic for the target cell type. Conversely, when the number of migrating cells is at or below background, the candidate chemotaxin is considered not chemotactic for the target cell type.

One suitable apparatus for carrying out *in vitro* chemotaxis assays is a 96-well ChemoTx® microplates (Neuroprobe Inc. Gaithersburg MD). The ChemoTx® instrument has a 96 well microplate of injection-molded from tissue-culture grade, transparent polystyrene. The microplate provides bottom wells for chemoattractants and other reagents. In place of top "wells," a framed filter is used that confines each cell-suspension sample to its site on top of the filter. The 96 sites on the filter correspond to the 96 wells in the microplate and cell suspension pipetted directly onto the sites on the top side of the filter sit in hemispherical drops during incubation. After incubation the migrated cells on the filter and in the microplate are counted. See, U.S. Pat. No. 5,284,753.

To assay for chemotactic activity using an apparatus such as the ChemoTx® microplates apparatus, candidate chemotaxins are added to lower wells (e.g., about 30 microliters) at one or more concentrations (e.g., about 1 nM, about 10 nM, about 100 nM, about 10 ng/ml, about 100 ng/ml and/or about 1 ug/ml), and cells are placed on porous polycarbonate filters positioned above the chemotaxin solution in each well. The filters have a pore size of about 3 um, or about 5 um, so that only cells exposed to an agent chemotactic for the cell will migrate into and/or through the filter. In the microplate configuration, about 20 microliters of cells (at about 1 x 10⁶ to about 1 x 10⁷ cells per ml, e.g., 5 x 10⁶ cells per ml) are usually used. The cells are incubated for a period of time (e.g., 0.5 to 6 hours, usually about 1.5 h, at from about 22°C to about 39°C, usually 37°C) and the cells are allowed to migrate into or through the filter.

When *in vitro* assays are conducted using a purified cell population (e.g., dendritic cells, neutrophils, immature dendritic cells, etc.) the assay conditions are usually tailored to the cell type being studied. For example, cells that are relatively plastic are able to crawl through certain filters, where as others become "stuck" in the filter. Thus, monocytes exposed to a monocyte chemotactic agent can migrate though a 5 um filter (into the lower chamber supernatant) which, in contrast, immature or mature dendritic cells exposed to a chemotactic agent migrate into a 3 um or 5 um filter, but are retained in the filter (i.e., the cells do not migrate through the filter). Therefore, the choice of migration assay format and the method of quantification can vary depending on the type of cells studied. For example, a 5 um pore size is usually used for a monocyte migration assay; the assay is typically carried out for 90 minutes, and the migrating cells (if any) are detected in the well. For mature dendritic cells, a 3 um pore size filter and 90 min incubation are used, and the cells are detected in the filter. These and other exemplary assay conditions are shown in Table 1. However, it will be appreciated that, as noted *supra,* a number of different assay formats and conditions can be used to determine that an agent does (or does not) have chemotactic activity (e.g., for a particular cell). Table 1 also provides exemplary positive and negative controls for use in assays. Positive controls are agents that, when used at 100 nM concentration, have chemotactic activity. Negative controls are agents that, when used at 100 nM concentration, do not have chemotactic activity.

**Table 1**

| Exemplary Assay Parameters and Positive and Negative Controls for In Vitro Chemotaxis Assays | | | | | |
|---|---|---|---|---|---|
| Cell Type | filter | time | location | + con. | - con. |
| monocyte | 5um | 90 | well | RANTES | MIP1b |
| immDC | 5um | 90 | filter | RANTES | SLC |
| matDC | 3um | 90 | filter | SLC | RANTES |
| neutro | 3um | 60 | well | IL8 | SLC |
| eosino | 3um | 60 | well | Eotaxin IL8 | |
| T cell | 3um | 180 | well | MDC | Eotaxin |
| B cell | 3um | 180 | well | SLC | Eotaxin |
| Macrophage | 5um | 90 | filter (+well) | RANTES | SLC |
| Key: +con. (positive control), -con. (negative control) are used at 100 nM concentrations. | | | | | |

The number of cells that have migrated in the presence of the agent can be determined using a variety of methods. To assay cells (e.g., dendritic cells) that are caught in a filter, the filters are scraped to remove nonadherent cells (i.e., cells that have simply settled onto the top side of the filter) and the cells that have moved into the filter or adhered to its lower surface are quantitated. For cells that are able to migrate through the filter (e.g., monocytes) the filter can be discarded and the number of cells that have migrated through the filter into the "lower" (chemotaxin-containing chamber) is determined. Suitable cell quantitation methods are well known and include direct (microscopic) counting of migrated cells, histological, cytochemical, immunofluorescence (using antibodies to cell-specfic or stage-specific markers), and *in situ* staining methods, use of radiolabled cells, and assays for RNA or DNA content (e.g., using reagents such CyQuant, Molecular Probes, Eugene OR, e.g., on a lysed cell extract), protein content (e.g., using stains such as Hema3), enzyme activity (e.g., β-glucuronidase), and the like. Often it is convenient to select stains that can be detected using densitometric or fluorescence plate readers.

In some embodiments, several concentrations of the candidate chemotaxin are used to detect an active concentration, e.g., at least two, typically at least three different concentrations, over a range of at least 10-fold, typically at least 100-fold, frequently at least 1,000-fold, and often at least 10,000-fold differences.

The chemotaxis assays can be carried out using a heterogeneous mixture of cells (e.g., peripheral blood mononuclear cells (PBMCs)) or a purified subpopulation (e.g., immature DCs, mature DCs, neutrophils, monocytes and the like) or tissue culture cells derived from a certain cell type and with characteristics of those cells (e.g., THP1 (an acute monocytic leukemia cell line, CEM acute lyphoblastic leukemia, T lymphoblast cell line). When a heterogeneous mixture of cells is used, the cell type or developmental stage of migrating cells is usually determined (e.g., based on morphology, histology, or staining of specific markers). When the assay is carried out using a homogeneous preparation (e.g., substantially purified neutrophils), it is not necessary to determine the cell type of migrating cells because any cells that migrate to the chemotaxin can be presumed to be of that type.

In various embodiments, a candidate chemotaxin is considered chemotactic for a particular cell type if the candidate chemotaxin, at a concentration about between about 1 pM and about 1 uM, e.g., between about 1 nM and 500 nM, e.g., 1 nM, about 10 nM, about 100 nM, or between about 1 pg/ml and about 10 ug/ml , e.g., between about 1 ng/ml and 1 ug/ml, e.g., about 10 ng/ml, about 100 ng/ml or about 1 ug/ml, attracts the cell at least 2-fold, preferably at least 4-fold, and often at least 8-fold more than "chemotaxis buffer" (a negative control) in an *in vitro* assay. Chemotaxis buffer is 0.1% BSA (Sigma) in HBSS (Life Technologies), with 1.4 mM Ca⁺⁺ and 1 mM Mg⁺⁺. HBSS is Hank's Balanced Salt Solutions (CaCl₂ (0.14g/l), KCl (0.4g/l), KH₂PO₄ (0.06g/l), MgCl₂-6H₂0 (0.1g/l), MgSO₂-7H₂0 (0.1g/l), NaCl (8g/l), NaHCO₃ (0.3g/l), Na₂HPO₄ (0.048g/l), D-glucose (1g/l)). An alternative negative control is PBS. A candidate chemotaxin is considered not to be chemotactic for a cell type if the candidate chemotaxin, at a concentration 1 nM, 10 nM, 100 nM, or 10 ng/ml, 100 ng/ml or 1 ug/ml, does not attract more cells (e.g., at least as many, sometimes at least 1.5-fold as many) as the negative control in an *in vitro* assay. Similarly, in various embodiments, a candidate chemotaxin is considered chemotactic for a particular cell type if the candidate chemotaxin, when injected at 2 ug, alternatively 10 ug, often 20 ug, attracts the cell at least 2-fold, preferably at least 5-fold, and often at least 10-fold more than PBS (a negative control) in an *in vivo* assay (e.g., intradermal injection into a mouse or monkey). In various embodiments, a candidate chemotaxin is considered not chemotactic for a cell type if the candidate chemotaxin, when injected at 2 ug, alternatively 10 ug, often 20 ug, does not attract more cells of the type (e.g., at least as many, sometimes at least 1.5-fold as many) that the negative control in an *in vivo* assay. PBS is phosphate buffered saline (KCI (0.2g/l), KH₂PO₄ (0.2g/l), NaCl (8.0g/l), Na₂HPO4 (2.16g/l)).

In some embodiments, an agent is determined to be a chemotactic agent because it has greater chemotactic activity than a known chemotaxin (e.g., known in the art or determined by the assays described herein).

Non-filter based chemotaxis assays may also be used, e.g., a cell migration assay can be carried out using a monolayer of cells grown on the filter as a barrier. In other examples, cellular motility may also be assessed by monitoring the movement of a single cell under a video microscope. In these types of experiments, the chemoattractant is applied through capillary, and the physical distance of cell's lateral movement toward the source of the chemoattractant is recorded.

### b) In vivo assays

In one embodiment, the chemotactic properties of an agent can be determined in animals, e.g., mammals such as non-human primates and mice, as described in Examples 2-4, *infra.* In one *in vivo* assay, the candidate chemotactic agent (e.g., 2-20 ug in PBS) is administered by intradermal injection to the animal. After a period of time (e.g., 24 h, 72 h, 96 h), the animal is euthanized, or a biopsy is taken. The area around the injection site is excised and subjected to routine histology or immunohistology techniques to determine the presence or absence of cell infiltration and, if an infiltrate is present, to characterize and quantitate the infiltrating cells (see, e.g., mononuclear cells, neutrophils, dendritic cells, etc.). For general histological methods, see, e.g., THE MANUAL OF HISTOLOGIC STAINING METHODS OF THE ARMED FORCES INSTITUTE OF PATHOLOGY", by Lee G. Luna, McGraw-Hill, 3rd edition, 1968 (hereinafter "Luna"). In one embodiment, cells are characterized by preparing frozen sections and staining with cell type specific antibodies, or cell type-specific combinations of antibodies, using well known methods (see, Harlow et al., 1988, Antibodies: A Laboratory Manual, Cold Spring Harbor Laboratory).

### 2. Characterization of Cell Types and Preparation Purified Cells

As noted *supra,* enriched or substantially purified cell populations can be used in *in vitro* chemotaxis assays. These cell populations can be prepared by a variety of methods known in the art, depending on the specific cell-type desired. Typically, substantially purified cell populations are prepared by culture under specific conditions, by physical characteristics such as behavior in a density gradient, by sorting according to characteristic markers (e.g., by fluorescence activated cell sorting (FACS) using monoclonal antibodies to cell-surface proteins, immunoprecipitation), or other methods.

Cells (e.g., in an enriched sample in *vitro* or in an infiltrate *in vivo*) can be identified histologically (see, e.g., Luna, *supra),* by immunological staining, and similar methods (see, e.g., Harlow, *supra;* Coligan et al., *supra*). Table 2A and 2B list exemplary markers useful for characterizing or purifying (e.g., FACS sorting) certain immune system cells. Many other markers are known in the art, both for the cells listed and for other immune system cells such as B-cells, T-cells, neutrophils, eosinophils, and others (see, e.g., Janeway-Travers, 1994, IMMUNOBIOLOGY Garland Pub., N.Y.; Paul, FUNDAMENTAL IMMUNOLOGY 3rd Ed, Raven Press N.Y. 1993). Table 2A shows certain classical cell surface markers; Table 2B shows certain chemokine receptors expressed by specific cell types.

**Table 2A**

| Selected Cell Surface Markers | | | | | | | | | | |
|---|---|---|---|---|---|---|---|---|---|---|
| | CD3 | CD14 | CD20 | CD25 | CD68 | CD80 | CD83 | C086 | HLA-DR | CD1a |
| Monocyte-derived Immature DCs | - | - | - | - | + | - | - | - | low | high |
| Mature DCs | - | - | - | + | | + | + | high | high | + |
| Macrophages | | high | | | + | | | | | - |
| Monocytes | - | + | - | - | | - | - | low | low | - |
| Key: Table 2A shows the levels of markers as determined by staining with an antibody specific for the marker. "-"indicates staining equivalent to an isotype control antibody (an isotype control antibody is an antibody of the same isotype as the staining antibody, but which does not recognize the epitope in question); "+" indicates at least 10 fold higher levels of staining as isotype control; "low" indicates staining 2-5 fold higher than isotype control; "high" indicates staining 10 to 1000 fold higher than isotype control. | | | | | | | | | | |

**Table 2B**

| | CC | CC | CC | CC | CC | CC | CXC | CXC | CXC | CXC | CXC | XC |
|---|---|---|---|---|---|---|---|---|---|---|---|---|
| | R1 | R2 | R3 | R5 | R6 | R7 | R1 | R2 | R3 | R4 | R5 | R1 |
| Immature DCs | + | - | - | + | - | - | - | - | - | + | - | - |
| Mature DCs | - | - | - | - | - | + | - | - | - | + | - | - |
| Monocytes | + | + | - | - | - | - | - | - | - | + | - | - |

To further assist the reader, methods for preparing substantially purified cell compositions for use in *in vitro* chemotaxis assays are briefly described *infra* and in the Examples. However, it will be recognized the invention does not require that any particular purification method be used, so long as the desired cells are obtained, and that many variations and alternative methods are known to those of skill in the art. Further, it will be appreciated that many other purification and detection methods, including methods suitable for cells not specifically listed herein, are known in the art or can be developed. Still further, it will be appreciated that, if desired, cloned cell lines derived from immune system tissues can be used in the chemotaxis assays described herein. General immunological, purification and cell culture methods are described in Coligan et al., 1991, CURRENT PROTOCOLS IN IMMUNOLOGY, John Wiley & Sons, including supplements through 1999. Unless otherwise specified, cells, when cultured, are incubated at 37°C in 5% CO₂.

### A) Monocytes

Suitable methods for purification of monocytes are found in Bender et al., 1996, *J. Immunol. Methods* 196:121-35 (also see U.S. pat. no. 5,994,126). Briefly, monocytes are isolated from PBMC by depletion of T cells using immobilized antibodies against a pan T cell surface marker CD2. Conveniently, a commercially available source of CD2 antibodies attached to magnetic beads (Dynal) is used. PBMC isolated from a buffy coat (typically 35 mls containing 400 x 10⁶ PMBC) by the conventional Ficoll gradient centrifugation method are resuspended in MACS buffer (made up of DPBS (HyClone) with 1% BSA (Sigma)) at 20 x 10⁶ cells per ml. DPBS is Dulbecco's Phosphate Buffered Saline (CaCl₂ (0.1g/l), KCl (0.2g/l), KH₂PO₄ (0.2g/l), MgCl₂-6H₂O (0.1g/l), NaCl (8.0g/l), Na₂HPO4 (2.16g/l)). An appropriate amount of immobilized CD2 + magnetic beads (typically 10 ul per 10⁶ cells) are added to the cells. The mixture is incubated for 15 minutes at 4°C with gentle rotation. The magnetically tagged T cells are removed from the unlabeled cells on a magnetic cell sorter (Dynal) according to the manufacturer's protocols. The unlabeled cells contain primarily monocytes and B cells.

B cells in the above preparation are removed by the conventional plastic adherence method. Briefly, PBMC depleted of T cells are allowed to adhere to the plastic of a T-175 tissue culture flask (100 x 10⁶ cells/ flask) (Costar) for 3 hours at 37°C. Non-adherent cells (comprising largely B cells) are aspirated. The flasks are rinsed 3 more times with DPBS to completely remove non-adherent cells. The resulting cells are largely enriched (i.e., > 90%) for monocytes.

Monocytes can also be isolated by positive selection of CD14 antigen. Briefly, PBMC isolated from peripheral blood, such as a buffy coat, by the standard Ficoll gradient centrifugation method are resuspended in MACS buffer at 1 x 10⁶ cells/ml. Immobilized antibodies against the CD14 surface antigen, such as CD14+ magnetic microbeads (Milteyni) are added (1 ul of beads per 1x10⁶ cells) and the mixture is incubated at 4°C for 15 minutes. Monocytes are separated from the other cell populations by passing the mixture through a positive selection column on a magnetic cell sorter (Miltenyi) according to manufacturers protocol. Monocytes that are retained on the column are eluted with MACS buffer after the column is removed from the MACS apparatus. Cells are then pelleted by centrifugation and resuspended in RMPI plus 10% FCS media at 10⁶ cells per ml. Monocytes isolated by this method are cultured essentially the same way as those isolated by the CD2+ depletion method.

### B. Preparing Purified Dendritic Cell Populations

Suitable methods for purification of dendritic cells, including separate mature and immature populations, are known in the art. Substantially purified dendritic cells (including subpopulations of mature or immature cells) can be prepared by selective *in vitro* culture conditions.

Dendritic cells are widely distributed in all tissues that have contact with potential pathogens (e.g., skin, gastrointestinal and respiratory tracts, and T cell-rich areas of the secondary lymphoid tissues). In the skin and upper respiratory tract they form a lattice of highly arborised cells (called Langerhans cells in the skin). After capturing antigen, dendritic cells in the peripheral tissues such as the skin and gut, traffic via the draining lymphatics to the T cell areas of lymph nodes where they present the antigen that was internalized at the point of pathogen contact. Immature dendritic cells function to take up and process antigens. During subsequent migration to the draining lymph node, the DC matures. The mature dendritic cells functions as the key APC to initiate immune responses by inducing the proliferation of pathogen specific cytotoxic and helper T cells.

Substantially pure populations of dendritic cells can be produced by *in vitro* culture (see, *infra).* In addition, there are marked changes in expression of chemokine receptors during dendritic cell maturation which can be used to identify cell stage (Campbell et al., 1998, *J Cell Biol* 141:1053; Chan et al., 1999, *Blood* 93:3610; Dieu et al., 1998, *J Exp Med* 188:373; Kellermann et al., 1999, *J Immunol* 162:3859). For example, immature dendritic cells express predominately CCR1, CCR5, and CXCR4. Upon maturation, these receptors, with the exception of CXCR4, are down regulated. *See also,* Table 2B.

In culture, immature forms of dendritic cells undergo maturation thought to be analogous to what occurs during the migration of dendritic cells from the point of antigen contact until their residence in the secondary lymphoid tissues. Human or macaque dendritic cells of various developmental stages can be generated in culture, from CD14⁺ blood progenitors using specific cytokines. A separate lineage of dendritic cells can be differentiated from CD34+ precursor cells from cord blood or bone marrow. Thus, in one embodiment of the invention, subpopulations of dendritic cells are generated for *in vitro* assays for identification of chemotactic compositions (i.e. to assess chemotaxin potency and selectivity against defined DC sub-types). Exemplary subpopulations of dendritic cells are: 1) immature peripheral blood monocyte derived cells; 2) mature peripheral blood monocyte derived cells, and 3) cells derived from CD34+ precursors. Subpopulations are isolated or produced by a variety of methods known in the art.

For example, immature and mature dendritic cells from PBMCs are produced according to Bender et al. *supra.*

### Immature Dendritic Cells

Briefly, PBMCs are depleted of T cells using immobilized antibodies against the cell surface marker CD2 (present on all T cells). Commercially available CD2+ dynabeads (Dynal) can be used according to manufacturer's protocol. The T-cell depleted mixture is separated into adherent versus non-adherent fractions by incubation of the cells on tissue culture grade plastic for 3 hours at 37°C. Adherent cells attach to the surface within the 3 hours while the non-adherent cells remain in suspension. Non-adherent cells are gently removed and adherent cells (generally CD14⁺ monocytes) are placed in culture media (e.g., RMPI + 10% FCS) supplemented with 1000 U/mL each of GM-CSF and IL-4 (R&D Systems, Minneapolis, MN) ("Day 1"). Between days 3-7 the cells begin to display a veiled morphology, and cytokines are replenished on days 2, 4, and 6, at which time the cells can be harvested as immature dendritic cells. In one embodiment, cells of this stage *in vitro* are isolated and used in the assay. Approximately 10 x 10⁶ dendritic cells are typically obtained from 400 x 10⁶ PBMCs.

Day 7 immature dendritic cells exhibit the typical dendritic cell morphology, with elongated cell body and many processes. The size of the cells increase significantly compared to the precursor monocytes. Immature dendritic cells can be characterized phenotypically by monitoring their expression of cell surface markers.

### Mature Dendritic Cells

Immature dendritic cells (generated from peripheral blood monocytes or from bone marrow derived CD34+ precursors), can be further activated and differentiated to become mature dendritic cells. Two methods are primarily used: MCM (macrophage conditioned medium) and double-stranded RNA-ploy (I:C) stimulation (Cella et al, 1999, J *Exp Med.* 189:821-9; Verdijk et al., 1999, J *Immunol*. 1999 1:57-61).

In the MCM method, day 6 immature dendritic cells are harvested by centrifugation and resuspended in at 10⁶ cells/ml in maturation medium (e.g., MCM diluted (up to 1:1 with RPMI containing 10% FCS). GM-CSF (1000 U/ml) and IL-4 (1000 U/ml) are added. Cells are cultured for three more days, without further addition of GM-CSF (1000 U/ml) and IL-4. Day 9 cells are used as mature dendritic cells.

In the poly (I:C) method, day 6 immature dendritic cells are harvested and resuspended in the standard culture medium (RPMI plus 10% FCS) supplemented with 20 ug/ml of poly (I:C) (Sigma), 1000 U/ml of GM-CSF and IL-4. Cells are cultured for another three days without additional cytokines. Day 9 cells are mature dendritic cells.

Mature dendritic cells generated by these two different methods exhibit phenotypic and functional properties distinct from those of immature dendritic cells or the precursor monocytes (See Table 2A). Mature dendritic cells from each preparation are thoroughly characterized by FACS to ensure that the desirable cell types are obtained.

Notably, mature dendritic cells generated express significantly higher level of MHC class II on the cell surface than immature cells. Expression of CD 80, CD83 and CD86 are also up-regulated. Chemokine receptor expression also changes dramatically during the maturation process. For instance, CCR1, CCR5 are down-regulated sharply in mature cells, while CCR7 is up-regulated and appears on the cell surface within a few hours after addition of MCM. Functionally, mature dendritic cells are no longer capable of taking up antigen efficiently, but gain the ability to stimulate the proliferation of naïve T cells and B cells. Mature dendritic cells also changes their migratory behaviors; they no longer respond to ligands of CCR1, CCR2, CCR5 such as MIP-1α, RANTES and MIP-1β. Instead, they respond to CCR7 ligands SLC and ELC.

### MCM Media

MCM is prepared by as described by Romani et al., 1996, *J. Immunol. Methods* 196:137) with minor modifications. Briefly, petri dishes (100 mm, Falcon) are coated with 5 ml of human Ig (10 mg/mL) for 30 min at 37°C and washed with PBS 2-3 times immediately before use. 50 x10⁶ PBMC in 8 ml volume are layered onto human Ig-coated plates for 1-2 hours. Non-adherent cells are washed away and discarded. The adherent cells are incubated in fresh complete medium (RPMI + 10% normal human serum) at 37°C and the resulting media (MCM) is collected after 24 hours. The TNF-a concentration in the MCM is determined by the standard ELISA method (e.g., using a TNF-a ELISA kit (R&D Systems, Minneapolis, MN)). The final TNF-a level in MCM is adjusted to 50 U/ml by mixing an appropriate amount of MCM with RPMI with 10% fetal calf serum.

### C) Neutrophils

Suitable methods for purification of neutrophils are known in the art. According to one suitable method, to purify neutrophils, whole fresh blood (WB) is diluted 1:1 with 3% dextran in a 50 ml centrifuge tube, and allowed to sediment for 30 - 45 m at room temperature. 25 ml of WB plus 25 ml dextran results in approximately 35 ml of supematant after 30 min sedimentation. The supernatant is layered over 12-15 ml Ficoll and centrifuged at 400 x g for 30-40 min at 18-20°C. The plasma/platelet layer containing mononuclear cells and Ficoll-Paque are removed by aspiration. Neutrophils are found in the white layer above the erythrocyte (RBC) layer. (In some preparations, the neutrophil and erythrocyte layers are mixed. In these cases RBCs are removed by hypotonic lysis in the following manner: 12.5 ml of cold 0.2% NaCl is added to the neutrophils/RBC pellet while vortexing. 12.5 ml of cold 1.6% NaCl is Immediately added while still vortexing. The cells are centrifuged at 60- 100 x g for 10 m and recovered. If necessary the lysis step is repeated.) The resulting neutrophils are >95% pure (with the eosinophis as the primary remaining cells).

### D) Macrophages

Suitable methods for purification of macrophages are known in the art. One suitable method is described in Paluka et al., 1998, "Dendritic cells as the terminal stage of monocyte differentiation," *J Imm*. 9:4587-95, which is incorporated herein in its entirety for all purposes.

### E) T Cells

Suitable methods for purification of T cells are known in the art. T lymphocytes are routinely prepared by removal of monocytes from the PBMC prepared by the standard Ficoll gradient centrifugation method (Coligan, *supra*). Removal of monocytes is achieved by allowing PMBC to adhere to the tissue culture flasks. The non-adherent cells (lymphocytes) are cultured in RPMI + 10% FCS in the presence of a combination of PHA (5 ug/ml) and human recombinant IL-2 (20 ng/ml) for two weeks, and the cells are harvested for assay.

### F) B Cells

Suitable methods for purification of B cells are known in the art. Highly purified B cell populations are isolated by negative selection with sequential depletion of monocytes/natural killer cells and T cells (as described in Current Protocols in Immunology). Depletion of monocytes and NK cells are carried out by using L-leucine methyl ester (L-LME). Briefly, PBMC isolated from peripheral blood (e.g, from a buffy coat) by the standard Ficol gradient method are resuspended at 3x10⁶ cells/ml in PBS. Freshly prepared L-LME (0.05 M solution in RPMI, no serum) is added to cells at 1:10 dilution (5 mM final). The mixture is incubated for 35 minutes at room temperature, the cells are pelleted by centrifugation, followed by washing 3x with PBS. T cells are further depleted by rosetting with neurominidase-treated sheep red blood cells (NSRBC) in the following manner: Cells are adjusted to 10⁷ cells/ml in RPMI/10% FCS. 5 ml of cells are transferred into a 50 ml centrifuge tube. 2.5 ml of fetal calf serum and 2.5 ml of NSRBC are added to the tube. The mixture is incubated for 10 minutes at 37°C. The cells are then centrifuged at 150xg at room temperature for 10 minutes to pellet cells and promote rosette formation. The mixture is incubated at 4°C for 2 hours. The pellet is gently resuspended, and the cell suspension is underlaid with Ficol (10ml). The tube is centrifuged at 400xg for 25 minutes. B cells in the interface are removed, pelletted and washed three times with HBSS. After repeating the rosetting step, B cells are resuspended in RPMI/10% FCS for migration assays.

### G) Eosinophils

Suitable methods for purification of eosinophils are known in the art. One method for preparation of substantially purified eosinophils is by further isolation from the preparation described in the neutrophil isolation protocol, *supra.* Separation of eosinophils form neutrophils is achieved by a negative selection method, where immobilized antibodies against the CD16 surface antigen are used to deplete the CD16 positive neutrophils. Briefly, the neutrophil preparation is resuspended in MACS buffer (1% BSA in DPBS) at a density of 10⁶ cells/ul. An equal volume of CD16+ microbeads (CD16 immobilized on magnetic spheres; Miltenyi Biotech; Auburn, CA) is mixed with the cells. The mixture is incubated at 4°C with gentle shaking for 30 minutes. Cells are then passed through a negative selection column that removes the magnetically labeled neutrophils (CS column, Miltenyi Biotech; Auburn, CA) LC, Miltenyi) and the column is washed with one column volume of MACs buffer. The flow-through and wash fractions contain eosinophils and are combined. Eosinophils isolated by this method are more than 95% pure (as determined by FACS, e.g., for the presence of the CD16+ cells).

### 3. Chemokines as Chemotactic Compositions

As discussed *supra,* the APC-chemotaxins may be any of a number of types of compounds. Often, the chemotaxin is a protein, such as a chemokine, or a protein mimetic. Thus, a chemotactic composition comprises one or more chemokines, chemokine analogues, or chemokine-encoding polynucleotides.

Chemokines are protein hormones that, among other activities, direct trafficking of white blood cells populations, e.g., in the primary lymphoid organs, blood, tissues, secondary lymphoid organs, lymph, and (in some instances), back into circulation. Over 50 distinct chemokines have been identified to date in humans, and numerous chemokines from other mammals and chemokines encoded by mammalian viruses are known.

Structurally, known chemokines are divided into four classes: CC, CXC, C, and CX3C, based on the number and spacing of the amino-terminal cysteine residues in a conserved structural motif. Chemokines exert promigratory effects by binding to an array of cell surface receptors on the surface of target leukocytes. Known receptors are of the seven transmembrane spanning, G protein coupled receptor (7TM GPCR) class. Of the almost 20 human chemokines receptors characterized (i.e., those receptors for which a ligand has been identified and binding and/or signaling events well characterized), nine are CC chemokine receptors (CCR), six are CXC chemokine receptors (CXCR), one is a CX3C chemokine receptor (CX3CR), and one is a C chemokine receptor (provisionally 'XCR'). In addition, one promiscuous chemokine receptor of broad binding specificity, originally known as the Duffy blood group antigen (Duffy Ag, sometimes denoted 'DARC') is known.

Chemokine proteins can be obtained from suppliers, e.g., R&D Systems (Minneapolis, MN; http://cytokine.rndsystems.com/cyt_cat/cyt_cat.html), or may be prepared using routine techniques based on published sequences (e.g., as described in Sambrook *et al*., 1989, MOLECULAR CLONING: A LABORATORY MANUAL, (2nd ed.) Vols. 1-3, Cold Spring Harbor Laboratory; Ausubel et al., 1999, Current Protocols In Molecular Biology, Greene Publishing and Wiley-Interscience, New York). For recent reviews on chemokines, see Ward et al., 1998, *Immunity* 9:1-11 and Baggiolini et al., 1998, *Nature* 392:565-568, and the references cited therein. Also see the CFB (Cytokine Facts Book, 1994, Academic Press Ltd.), Chemokine Facts Book, 1997, Academic Press Ltd. and the GenBank protein sequence database http://www.ncbi. nim.nih.gov/entrez/query.fcgi). Additional references are provided *infra* in section 14.

The chemokines used in, or to prepare, the compositions of the present invention may be natural (i.e., have the sequence of a naturally occurring chemokine), may be the product of *in vitro* recombination of polynucleotides encoding naturally occurring chemokines, or may be synthetic (i.e., chemically synthesized) or recombinant variants of naturally occurring chemokine sequences. Chemokines comprise sequences from human, primate, rodent, viral, and other species. In some embodiments, xenogeneic sequences are used in the vaccination methods of the invention (e.g., if the most potent chemokine is from a species other than the species of the subject being immunized) because any immunogenic effects will likely enhance the adjuvant activity.

### 4. Exemplary Chemokine Compositions

In the present invention, the compositions contain at least one APC-chemotaxin, selected from the chemokines vMCK-2 and mC10, variants thereof that do not change the chemotatic properties of the polypeptide, or a polynucleotide encoding one of the above.

*In vitro* chemotaxis assays were carried out to determine the chemotactic profile of a comprehensive set of known chemokines. A number of known chemokines are chemotactic for immature but not mature dendritic cells, including: hMIP1α, hMIP1α (70aa), mMIP-1α, hRANTES, hMET-RANTES, mRANTES, hHCC-1, hMPIF-1, hMPIF-1 (22-137), hMPIF-1 (46-137), hMIP-1δ, hMCP-4, mMCP-5, mMARC, mEotaxin, mMCP-1(JE), mTECK, mMIP-2, mBLC, hLeukotactin, mMIG, and mMIP-1β. Other particularly useful chemokines include hMCP-2, hMCP-3, vMIP-1, hMIP-3α, hMIP-3β, and vMCK-2 (see Examples, *infra*). Several chemokines were chemotactic for immature dendritic cells but were not chemotactic for neutrophils and other cell types tested in in vitro assays (mC10, mMDC, hMIP-1β, mMIP-1γ; see Table 3).

**Table 3**

| | Immature DCs | Mature DCs | Monocytes | Neutrophils | Eosinophils | CEM (T-Cell line)¹ |
|---|---|---|---|---|---|---|
| mC10 | + | - | - | - | - | - |
| mMDC | + | - | - | - | - | - |
| hMIP-1β | + | - | - | - | - | - |
| mMIP-1γ | + | - | - | - | - | - |

| | | | | | | |
|---|---|---|---|---|---|---|
| 1. ATTC No. CCL-119. | | | | | | |

### 5. Homologs and Variants of Naturally Occurring Chemokines

In one embodiment, an APC-chemotaxin molecule has the sequence of a naturally occurring chemotaxin molecule or has an amino acid sequence with substantial amino acid sequence identity to the sequence of a naturally occurring chemotaxin molecule. For example, chemokine polypeptides, such as the chemokines listed in *supra,* can be modified in ways that do not change the chemotactic properties of the naturally occurring polypeptide, for example, by conservative amino acid substitutions, truncations (especially at the termini), small internal deletions, insertions, and the like. Such modifications can be made using routine genetic engineering techniques, e.g., using site-directed mutagenesis, and the resulting mutants assessed for chemotactic properties (e.g., using the assays disclosed herein).

In addition, recombinant and synthetic techniques can be used to modify naturally occurring chemokine molecules or sequences (including, but not limited to those listed *supra* and in Table 3) to modify the chemotactic properties of the polypeptide compared to the parent polypeptide(s).

### A. Engineered chemokines

For the purposes of the present invention, synthetic, genetically engineered or recombinant chemotaxins can beprepared and assessed for the ability to mobilize APCs (e.g., dendritic cells). In one embodiment, APC-chemotaxins are generated from a combination of natural chemokines using synthetic or recombinant DNA technology. For example, different chemokines (e.g., human, viral, murine, and the like) can be recombined (e.g., genetically) to form chimeras or "hybrikines" that are tested for the desired activity (e.g., the ability attract immature dendritic cells but not neutrophils, enhanced chemoattractant activity at lower concentrations). In a related embodiment, the chimeras are chemically synthesized based on the sequence of parent (e.g., naturally occurring) chemokines.

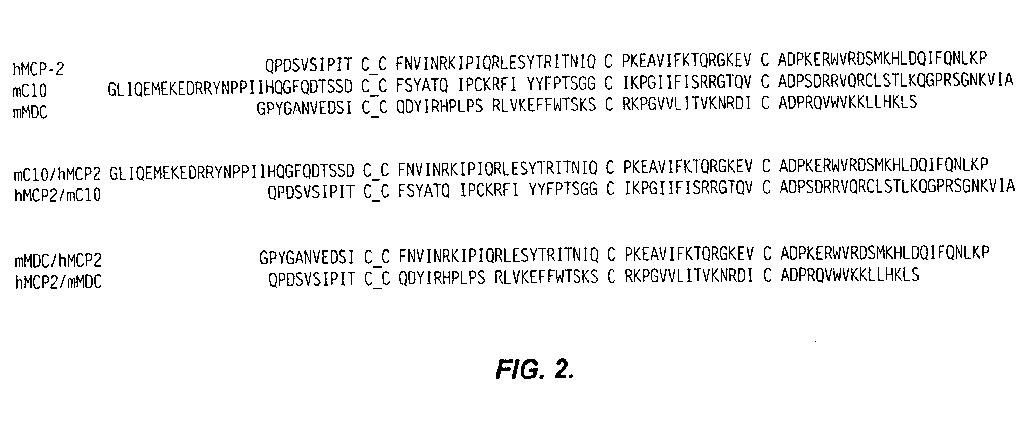

In one embodiment, the sequences of chemokine polypeptides of interest are divided into four 'domains' as dictated by the spacing of the cysteine residues (see Fig. 2 and Example 5). Alternatively, the sequences are divided into multiple "domains" (e.g., 2, 3, 4, or more) of any length (but typically at least 5, more often at least 10 residues), for the purpose of constructing hybrids. The sequences are conceptually recombined to form chimeric sequences, in which one region has a sequence of a first chemokine polypeptide and a second region has the sequence of a second chemokine polypeptide, as illustrated in Fig. 2. Chimeric polypeptides (hybrikines) having the chimeric sequence are then produced by routine synthetic means (or, alternatively, by using recombinant DNA techniques, as described *infra),* Polypeptide synthetic methods are well known in the art and are described in, e.g., U. S. Pat. No. 4,108,846; see also, Caruthers et al., 1980, *Nucleic Acids Res. Symp. Ser*., 215-223; Hom et al., 1980, *Nucleic Acids Res. Symp. Ser.,* 225-232; Roberge, et al., 1995, *Science* 269:202). If desired, short polypeptides may be fused by condensation of the amino terminus of one molecule with the carboxyl terminus of the other molecule to form a peptide bond to produce a longer polypeptide. The newly synthesized peptide can be substantially purified, for example, by preparative high performance liquid chromatography (e.g., Creighton, 1983, PROTEINS, STRUCTURES, AND MOLECULAR PRINCIPLES, W.H. Freeman and Co, New York NY).

In an alternate embodiment, the polynucleotides encoding parent chemokines of interest are manipulated to produce variant or chimeric chemokines such as those described *supra* (e.g., the polynucleotide is divided into multiple 'domains' as dictated by the spacing of the cysteine residues of the encoded polypeptide and hybrid gene constructs) or chemically synthesized polynucleotides are prepared. The hybrid genes are subcloned into appropriate expression vectors and introduced (e.g., transfected) into host cells (e.g., bacterial or eukaryotic cells) and the cells are cultured under conditions in which the recombinant protein is expressed. Supernatants of transfected cells are assayed for the desired chemoattractant properties using the assays described *supra.* Techniques for nucleic acid manipulation and expression are described generally, e.g., in Sambrook, et al., 1989, MOLECULAR CLONING: A LABORATORY MANUAL (2d ed.), vols. 1-3, Cold Spring Harbor Laboratory; and Ausubel, et al. (eds.) (supplemented to 1999) CURRENT PROTOCOLS IN MOLECULAR BIOLOGY, Greene and Wiley, NY.

In an embodiment, chimeric molecule can comprise at least 10 contiguous residues from each of at least two different naturally occurring chemokines. At least one of the naturally occurring chemokines, is selected from mC10 and vMCK-2. One, and sometimes tow or more of the further chemokines, can be selected from: hMIP1α, hMIP1α (70aa), mMIP-1α, hRANTES, hMET-RANTES, mRANTES, hHCC-1, hMPIF-1, hMPIF-1 (22-137), hMPIF-1 (46-137), hMIP-1δ, hMCP-4, mMCP-5, mMARC, mEotaxin, mMCP-1(JE), mTECK, mMIP-2, mBLC, hLeukoiactin, mMIG, and mMIP-1β, hMCP-2, hMCP-3, vMIP-1, hMIP-3α, hMIP-3β, vMCK-2, and especially, mC10, mMDC, hMIP-1β, and mMIP-1γ. In an embodiment the two different naturally occurring chemokines are from different species.

Derivatives of naturally occurring chemokines with enhanced dendritic cell attractant properties and decreased recruitment of non-dendritic immune cells, are constructed using different hybrids of human and viral chemokines. For example, an APC-chemotaxin (e.g. a chemokine) with potent dendritic cell chemotactic activity but undesired neutrophil chemotactic activity can be recombined with a polypeptide with weaker dendritic cell activity and no neutrophil chemotactic activity to produce a polypeptide with strong dendritic cell activity and no neutrophil chemotactic activity.

### B. APC-Chemotaxins Produced by Gene Shuffling Chemokine Polynucleotides

In one embodiment, derivatives of chemokines with enhanced properties (e.g., the ability attract immature dendritic cells but not neutrophils, enhanced chemoattractant activity at lower concentrations, and the like) are constructed using forced *in vitro* genetic evolution using any of several routine techniques, including error-prone PCR or recombination/gene shuffling approaches. Methods for generating new polypeptides with desired activities by gene "shuffling" are known in the art. Various shuffling methods are described in Patten et al., 1997, *Curr. Opin. Biotech.* 8:724-733; Stemmer, 1994, *Nature* 370:389-391; Stemmer et al., 1994, *Proc. Natl. Acad. Sci. USA* 91:10747-10751; Zhao et al., 1997, *Nucleic* Acids Res. 25:1307-1308; Crameri et al., 1998, *Nature* 391: 288-291; Crameri et al., 1997, *Nat. Biotech.* 15:436-438; Arnold et al., 1997, *Adv. Biochem. Eng. Biotechnol*. 58:2-14; Zhang et al., 1997, *Proc. Natl. Acad. Sci. USA* 94:4504-4509; Crameri et al., 1996, *Nat. Biotechnol.* 14:315-319; Crameri et al., 1996, *Nat. Med.* 2:100-102; PCT publications WO95/22625; WO97/20078; WO97/35957; WO97/35966; WO98/13487; WO98/13485; PCT 98/00852; PCT 97/24239; and U.S. Patent Nos. 5,605,793, 5,811,238, and 5,928,905. One method of gene shuffling conducting a polynucleotide amplification process on overlapping segments of a population of variants of a polynucleotide under conditions whereby one segment serves as a template for extension of another segment, to generate a population of recombinant polynucleotides, and screening or selecting a recombinant polynucleotide or an expression product thereof for a desired property. Some methods of shuffling use random point mutations (typically introduced in a PCR amplification step) as a source of diversity. The resulting polypeptides are tested for chemotactic activity as described above.

In an embodiment, the gene shuffling is carried out beginning with a polynucleotide encoding a particular chemokine (e.g., vMCK-2 or mC10). In a different embodiment, "family shuffling" is used (see, e.g., Cramer et al., 1998, *Nature* 152:88-91; Chang et al., 1999, *Nat Biotechnol* 17:793-7) and at least two "parental" chemokine-encoding polynucleotides are used in the family shuffling reaction. At least one of the parental chemokine-encoding polynucleotides encodes mC10, vMCK-2, or a species homolog thereof.

In a related embodiment, the second or further of the parental chemokine-encoding polynucleotides encode a chemokine from the group hMIP1a, hMIP1α (70aa), mMIP-1α, hRANTES, hMET-RANTES, mRANTES, hHCC-1, hMPIF-1, hMPIF-1 (22-137), hMPIF-1 (46-137), hMIP-1δ, hMCP-4, mMCP-5, mMARC, meotaxin, mMCP-1(JE), mTECK, mMIP-2, mBLC, mMIP-1γ, mMIG, and mMIP-1β, hMCP-2, hMCP-3, vMIP-1, hMIP-3α, hMIP-3β, mMDC, hMIP-1β, and hLeukotactin.

In an embodiment, the resulting shuffled ("evolved") molecule comprises at least 10 contiguous residues from at least one, often at least two different naturally occurring chemokines, e.g., such as the sets listed *supra.*

### C. Other APC-Chemotaxins Prepared by Mutation of Chemokine Molecules

In other aspects of the invention, a parental APC-chemotaxin (e.g., chemokine) is modified by conventional *in vitro* mutagenesis methods (e.g., site directed mutagenesis (Ausubel, *supra)* or *in vitro* genetic manipulation of chemokine-encoding polynucleotides. The activity of the resulting variant can be determined using the *in vitro* and *in vivo* assays described herein.

In another aspect of the invention, a parental APC-chemotaxin polypeptide (e.g., a chemokine) is modified (e.g., by *in vitro* genetic manipulation of the chemokine-encoding polynucleotide) to produce amino- or carboxy-truncated version of the mature protein. Alternatively, these truncated version of the APC-chemotaxin can be chemically synthesized, or produced by enzymatic processing of a naturally occurring chemokine. In addition, variants with conservative substitutions that retain the desired properties may be used in the methods and compositions of the invention. Conservative substitution tables providing functionally similar amino acids are well known in the art. For example, one exemplary guideline to select conservative substitutions includes (original residue followed by exemplary substitution): ala/gly or ser; arg/ lys; asn/ gln or his; asp/glu; cys/ser; gln/asn; gly/asp; gly/ala or pro; his/asn or gln; ile/leu or val; leu/ile or val; lys/arg or gln or glu; met/leu or tyr or ile; phe/met or leu or tyr; ser/thr; thr/ser; trp/tyr; tyr/trp or phe; val/ile or leu.

An alternative exemplary guideline uses the following six groups, each containing amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W); (see also, e.g., Creighton (1984) *Proteins,* W.H. Freeman and Company; Schulz and Schimer (1979) *Principles of Protein Structure,* Springer-Verlag).

### D. Polypeptide Mimetics

Polypeptide mimetics are also suitable for use in the methods of the invention. The terms "mimetic" and "peptidomimetic" refer to a synthetic chemical compound which has substantially the same structural and/or functional characteristics as an APC-chemotaxin polypeptide of the invention (e.g., a specific chemokine). The mimetic can be either entirely composed of synthetic, non-natural analogues of amino acids, or, is a chimeric molecule of partly natural peptide amino acids and partly non-natural analogs of amino acids. The mimetic can also incorporate any amount of natural amino acid conservative substitutions as long as such substitutions also do not substantially alter the mimetic's structure and/or activity. Polypeptide mimetic compositions can contain any combination of nonnatural structural components, which are typically from three structural groups: a) residue linkage groups other than the natural amide bond ("peptide bond") linkages; b) non-natural residues in place of naturally occurring amino acid residues; or c) residues which induce secondary structural mimicry, *i*.*e*., to induce or stabilize a secondary structure, e.g., a beta turn, gamma turn, beta sheet, alpha helix conformation, and the like.

A polypeptide can be characterized as a mimetic when all or some of its residues are joined by chemical means other than natural peptide bonds. Individual peptidomimetic residues can be joined by peptide bonds, other chemical bonds or coupling means, such as, e.g., glutaraldehyde, N-hydroxysuccinimide esters, bifunctional maleimides, N,N'-dicyclohexylcarbodiimide (DCC) or N,N'-diisopropylcarbodiimide (DIC). Linking groups that can be an alternative to the traditional amide bond ("peptide bond") linkages include, *e*.*g*., ketomethylene (e.g., -C(=O)-CH₂- for -C(=O)-NH-), aminomethylene (CH₂-NH), ethylene, olefin (CH=CH), ether (CH₂-O), thioether (CH₂-S), tetrazole (CN₄-), thiazole, retroamide, thioamide, or ester (see, *e*.*g*., Spatola (1983) in *Chemistry and Biochemistry of Amino Acids, Peptides and Proteins,* Vol. 7, pp 267-357, "Peptide Backbone Modifications, □"Marcell Dekker, NY).

A polypeptide can also be characterized as a mimetic by containing all or some non-natural residues in place of naturally occurring amino acid residues. Nonnatural residues are well described in the scientific and patent literature; a few exemplary nonnatural compositions useful as mimetics of natural amino acid residues and guidelines are described below.

Mimetics of aromatic amino acids can be generated by replacing by, *e.g.,* D- or L- naphylalanine; D- or L- phenylglycine; D- or L-2 thieneylalanine; D- or L-1, -2, 3-, or 4- pyreneylalanine; D- or L-3 thieneylalanine; D- or L-(2-pyridinyl)-alanine; D- or L-(3-pyridinyl)-alanine; D- or L-(2-pyrazinyl)-alanine; D- or L-(4-isopropyl)-phenylglycine; D-(trifluoromethyl)-phenylglycine; D-(trifluoromethyl)-phenylalanine; D-p-fluorophenylalanine; D- or L-p-biphenylphenylalanine; K- or L-p-methoxybiphenylphenylalanine; D- or L-2-indole(alkyl)alanines; and, D- or L-alkylainines, where alkyl can be substituted or unsubstituted methyl, ethyl, propyl, hexyl, butyl, pentyl, isopropyl, iso-butyl, sec-isotyl, iso-pentyl, or a non-acidic amino acids. Aromatic rings of a nonnatural amino acid include, e.g., thiazolyl, thiophenyl, pyrazolyl, benzimidazolyl, naphthyl, furanyl, pyrrolyl, and pyridyl aromatic rings.

Mimetics of acidic amino acids can be generated by substitution by, e.g., non-carboxylate amino acids while maintaining a negative charge; (phosphono)alanine; sulfated threonine. Carboxyl side groups (e.g., aspartyl or glutamyl) can also be selectively modified by reaction with carbodiimides (R'-N-C-N-R') such as, *e.g.,* 1-cyclohexyl-3(2-morpholinyl-(4-ethyl) carbodiimide or 1-ethyl-3(4-azonia- 4,4- dimetholpentyl) carbodiimide. Aspartyl or glutamyl can also be converted to asparaginyl and glutaminyl residues by reaction with ammonium ions.

Mimetics-of basic amino acids can be generated by substitution with, e.g., (in addition to lysine and arginine) the amino acids omithine, citrulline, or (guanidino)-acetic acid, or (guanidino)alkyl-acetic acid, where alkyl is defined above. Nitrile derivative *(e.g.,* containing the CN-moiety in place of COOH) can be substituted for asparagine or glutamine. Asparaginyl and glutaminyl residues can be deaminated to the corresponding aspartyl or glutamyl residues.

Arginine residue mimetics can be generated by reacting arginyl with, e.g., one or more conventional reagents, including, e.g., phenylglyoxal, 2,3-butanedione, 1,2-cyclohexanedione, or ninhydrin, preferably under alkaline conditions.

Tyrosine residue mimetics can be generated by reacting tyrosyl with, e.g., aromatic diazonium compounds or tetranitromethane. N-acetylimidizol and tetranitromethane can be used to form O-acetyl tyrosyl species and 3-nitro derivatives, respectively.

Cysteine residue mimetics can be generated by reacting cysteinyl residues with, e.g., alpha-haloacetates such as 2-chloroacetic acid or chloroacetamide and corresponding amines; to give carboxymethyl or carboxyamidomethyl derivatives. Cysteine residue mimetics can also be generated by reacting cysteinyl residues with, e.g., bromo-trifluoroacetone, alpha-bromo-beta-(5-imidozoyl) propionic acid; chloroacetyl phosphate, N-alkylmaleimides, 3-nitro-2-pyridyl disulfide; methyl 2-pyridyl disulfide; p-chloromercuribenzoate; 2-chloromercuri-4 nitrophenol; or, chloro-7-nitrobenzo-oxa-1,3-diazole.

Lysine mimetics can be generated (and amino terminal residues can be altered) by reacting lysinyl with, e.g., succinic or other carboxylic acid anhydrides. Lysine and other alpha-amino-containing residue mimetics can also be generated by reaction with imidoesters, such as methyl picolinimidate, pyridoxal phosphate, pyridoxal, chloroborohydride, trinitrobenzenesulfonic acid, O-methylisourea, 2,4, pentanedione, and transamidase-catalyzed reactions with glyoxylate.

Mimetics of methionine can be generated by reaction with, e.g., methionine sulfoxide. Mimetics of proline include, e.g., pipecolic acid, thiazolidine carboxylic acid, 3- or 4- hydroxy proline, dehydroproline, 3- or 4-methylproline, or 3,3,-dimethylproline. Histidine residue mimetics can be generated by reacting histidyl with, e.g., diethylprocarbonate or para-bromophenacyl bromide.

Other mimetics include, e.g., those generated by hydroxylation of proline and lysine; phosphorylation of the hydroxyl groups of seryl or threonyl residues; methylation of the alpha-amino groups of lysine, arginine and histidine; acetylation of the N-terminal amine; methylation of main chain amide residues or substitution with N-methyl amino acids; or amidation of C-terminal carboxyl groups. A component of a natural polypeptide can also be replaced by an amino acid (or peptidomimetic residue) of the opposite chirality. Thus, any amino acid naturally occurring in the L-configuration (which can also be referred to as the R or S, depending upon the structure of the chemical entity) can be replaced with the amino acid of the same chemical structural type or a peptidomimetic, but of the opposite chirality, generally referred to as the D- amino acid, but which can additionally be referred to as the R- or S- form.

The mimetics of the invention can also include compositions that contain a structural mimetic residue, particularly a residue that induces or mimics secondary structures, such as a beta turn, beta sheet, alpha helix structures, gamma turns, and the like. For example, substitution of natural amino acid residues with D-amino acids; N-alpha-methyl amino acids; C-alpha-methyl amino acids; or dehydroamino acids within a peptide can induce or stabilize beta turns, gamma turns, beta sheets or alpha helix conformations. Beta turn mimetic structures have been described, e.g., by Nagai (1985) *Tet. Lett.* 26:647-650; Feigl (1986) J. *Amer. Chem. Soc.* 108:181-182; Kahn (1988) *J. Amer. Chem.* Soc. 110:1638-1639; Kemp (1988) *Tet. Lett*. 29:5057-5060; Kahn (1988) *J. Molec. Recognition* 1:75-79. Beta sheet mimetic structures have been described, e.g., by Smith (1992) *J. Amer. Chem.* Soc. 114:10672-10674. For example, a type VI beta turn induced by a cis amide surrogate, 1,5-disubstituted tetrazol, is described by Beusen (1995) *Biopolymers* 36:181-200. Incorporation of achiral omega-amino acid residues to generate polymethylene units as a substitution for amide bonds is described by Banerjee (1996) *Biopolymers* 39:769-777. Secondary structures of polypeptides can be analyzed by, e.g., high-field 1H NMR or 2D NMR spectroscopy, see, e.g., Higgins (1997) J. *Pept. Res.* 50:421-435. See also, Hruby (1997) *Biopolymers* 43:219-266, Balaji, *et al.,* U.S. Pat. No. 5,612,895.

Specific examples of mimetics which can be incorporated into the polypeptide of the invention include those described by, *e.g,* Zhang (1998) Biochemistry 37:12465-12476, who constructed functionally active (R and S)-gamma-lactam conformational mimetics using a 3-(R or S)-amino- 2-oxo-1-pyrrolidine-acetamido moiety in place of the Pro-Gly of the tridecapeptide *Saccharomyces* cerevisiae alpha-factor mating pheromone. Brady (1998) *J. Med. Chem.* 41:401-406, used a resin-based route for the synthesis of thrombin inhibitor mimetic with a range of lipophilic carboxylic amides. Baures (1997) *J. Med. Chem*. 40:3594-3600, constructed a diketopiperazine conformational mimic into a L-prolyl-L-leucylglycinamide structure and into the bicyclic lactam PLG peptidomimetic structure of dopamine receptor. Beaulieu (1997) J. *Med..Chem.* 40:2164-2176, used a (hydroxyethyl)amidosuccinoyl core to synthesize a peptidomimetic structure to inhibit HIV viral protease activity. Misicka (1997) *J. Pept.* Res. 50:48-54, designed mimetics of deltorphin I and dermenkephalin containing stereoisomer of the unusual amino acid beta-methylphenylalanine to generate peptides with increased ligand binding specificity.

The skilled artisan will recognize that individual synthetic residues and polypeptides incorporating mimetics can be synthesized using a variety of procedures and methodologies, which are well described in the scientific and patent literature, e.g., Organic Syntheses Collective Volumes, Gilman et al. (Eds) John Wiley & Sons, Inc., NY. Polypeptides incorporating mimetics can also be made using solid phase synthetic procedures, as described, e.g., by Di Marchi, *et al*., U.S. Pat. No. 5,422,426. Mimetics of the invention can also be synthesized using combinatorial methodologies. Various techniques for generation of peptide and peptidomimetic libraries are well known, and include, e.g., multipin, tea bag, and split-couple-mix techniques; see, e.g., al-Obeidi (1998) *Mol. Biotechnol.* 9:205-223; Hruby (1997) *Curr. Opin. Chem. Biol.* 1:114-119; Ostergaard (1997) *Mol. Divers.* 3:17-27; Ostresh (1996) *Methods Enzymol*. 267:220-234.

### 6. Small Chemokine Mimetics

In an embodiment, small molecule chemokine mimetics are used to mobilize APCs. Typically, small molecule mimetics are identified using high throughput screening technologies for small molecule compounds that bind chemokine receptors (CRs) and transduce signal (e.g., calcium ion mobilization or other chemokine receptor-mediated responses). For example, small molecules can be screened initially for the ability to agonize chemokine receptors that are expressed on immature dendritic cells and/or are not expressed on other cells (see, e.g., Table 2B). The agonizing activity can be detected in a variety of ways, such as detecting Ca2+ mobilization responses on transfectant cells expressing cloned chemokine receptors known to be present on APCs.

The chemotactic properties of the molecules are then determined using the assays described *supra,* and those with desired specificity (e.g., chemotactic for immature but not mature dendritic cells) are used in the methods of the present invention.

### 7. Chemotactic Compositions

The chemotactic compositions of the invention contain one or more APC-chemotaxins (or chemotaxin-encoding polynucleotides). In an embodiment, the composition contains an APC-chemotaxin that is an isolated or recombinant polynucleotide or polypeptide. In an embodiment, the APC-chemotaxin(s) is/are the predominant species (i.e., greater than about 50%, more often greater than about 80% by weight of the total of the members of the class of molecule in the composition) of its class (e.g., polypeptide, polynucleotide, lipid, carbohydrate) in the composition. In other embodiments, the APC-chemotaxin(s) is "biologically pure." The words "isolated," "pure" "substantially purified" or "biologically pure" refer to material which is substantially or essentially free from components which normally accompany it as found in its native state. Thus, in an embodiment, the chemotactic compositions of the invention contains APC-chemotaxins free of materials normally associated with their *in situ* environment (if naturally occurring). Typically, the isolated, chemotaxins of the invention are at least about 80% pure, usually at least about 90%, and preferably at least about 95%. Protein purity or homogeneity may be determined by a number of means well known in the art, such as polyacrylamide gel electrophoresis of a protein sample, followed by visualization upon staining. For certain purposes high resolution will be needed and HPLC or a similar means for purification utilized.

In embodiments, the compositions may additionally contain an excipient or carrier, such as described *infra.* In all cases, the composition includes one or more antigens (i.e., the antigen to which it is desired to induce or enhance an immune response), as is discussed in greater detail *infra.* In embodiments, the compositions may contain a conventional adjuvant. Conventional adjuvants typically convert soluble protein antigens into particulate form. They often include bacteria or bacterial products. Exemplary conventional adjuvants include Freund's Incomplete Adjuvant, Freund's Complete Adjuvant, Merck Adjuvant 65, AS-2, alum, aluminum phosphate, mineral gels such as aluminum hydroxide, and surface active substances such as lysolecithin, pluronic polyols, polyanions, peptides, oil emulsions, keyhole limpet hemocyanin, and dinitrophenol. In some embodiments, the conventional adjuvant is an adjuvant suitable for use in human patients.

### 8. Antigens

The present invention provides a method of eliciting or enhancing an immune response to an antigen, e.g., a predetermined or specified antigen. An antigen is a molecule that reacts with an antibody. In some embodiments the antigen is an immunogen. In some embodiments the antigen is linked to a protein carrier. For example, in one embodiment of the invention, an APC-chemotaxin and an antigen are physically linked (e.g., made as a fusion protein, stably cross-linked using chemical crosslinkers, or linked via complexes such as biotin and streptavidin).

Typically, an antigen is a peptide, a polypeptide, chemical compound, microbial pathogen, bacteria (e.g., live, attenuated, or inactivated), a virus (including inactivated virus particles, modified live viral particles, and recombinant virus particles), a recombinant cell, glycoproteins, lipoproteins, glycopeptides, lipopeptides, toxoids, carbohydrates, tumor-specific antigens; and other immunogenic components of pathogens. In one embodiment, mixtures of two or more antigens are employed. In some embodiments, the antigen is biologically pure.

In one embodiment, the methods and reagents of the invention can be used to provide protection from exogenous foreign infectious pathogenic agents (such as bacteria, virus, and the like) prior to expected or possible exposure in a related embodiment, the methods and reagents of the invention can be used to provide therapeutic effects against exogenous foreign pathogens to which an individual has been exposed or to an individual displaying symptoms of exposure.

In one embodiment, reagents and methods of the invention can be used to treat cancers, including, but not limited to, melanomas, lung cancers, thyroid carcinomas, breast cancers, renal cell carcinomas, squamous cell carcinomas, brain tumors and skin cancers. In one embodiment, the antigen is a tumor associated antigen (tumor specific antigen). Tumor antigens are molecules, especially cell surface proteins, that are differentially expressed in tumor cells relative to non-tumor tissues (e.g., telomerase).

For prophylactic use, compositions containing the APC-chemotaxins are administered (e.g., in conjunction with antigens) to a subject susceptible to or otherwise at risk of a disease, e.g. a tumor, cancer, infection, and the like. For therapeutic use, compositions containing the APC-chemotaxins are administered (e.g., in conjunction with antigens) to a subject once a disease, e.g. a tumor, cancer, infection, and the like, is detected or diagnosed, or after surgical removal, e.g. of tumors.

Exemplary antigens or vaccine components of the invention include antigens derived from microbial pathogens such as bacteria [e.g., Pertussis (Bordetella pertussis, inactivated whole organism); Cholera (Vibrio cholerae, whole killed organism); Meningitis (Neisseria meningitidis, polysaccharide from organism); Lyme Disease (Borrelia burgdorferi, lipoprotein OspA); Haemophilus B (Haemophilus influenza B polysaccharide, Tetanus conjugate or OmpC); Pneumonia (Streptococcs pneumoniae capsular polysaccharide) Typhoid (Salmonella typhi polysaccharide vaccine, killed whole organism)], viruses including inactivated virus particles, modified live viral particles, and recombinant virus particles to influenza virus; Hepatitis A; Hepatitis B; Measles; Rubella virus; Mumps ; Rabies; Poliovirus; Japanese Encephalitis virus; Rotavirus; Varicella], Diphtheria (Corynebacterium diphtheriae) and Tetanus (Clostridium tetani).

### 9. Polynucleotide Chemotactic Compositions

In one aspect, the APC-chemotaxin, the antigen, or both are delivered as DNA such that the polypeptides are generated *in situ*. In one embodiment, the DNA is "naked," as described, for example, in Ulmer et al., *Science* 259:1745-1749, 1993 and reviewed by Cohen, 1993, Science 259:1691-1692. The uptake of naked DNA may be increased by coating the DNA onto a carrier, e.g. biodegradable beads, which is efficiently transported into the cells. In such vaccines, the DNA may be present within any of a variety of delivery systems known to those of ordinary skill in the art, including nucleic acid expression systems, bacterial and viral expression systems. Techniques for incorporating DNA into such expression systems are well known to those of ordinary skill in the art. See, e.g., WO90/11092, WO93/24640, WO 93/17706, and U.S. Pat. No. 5,736,524

### 10. Administration of APC-Chemotaxin and Antigen

The compositions of the present invention contain one or more antigens (or antigen-encoding polynucleotides). The antigens can be administered in combination with the APC-chemotaxin (i.e., in the same mixture). Alternatively, they can be administered separately.

Thus, in one aspect, the invention provides an immunization method in which a combination of one or more antigens (or antigen-encoding polynucleotides) and one or more APC-chemotaxins of the invention (or APC-chemotaxin-encoding polynucleotides) are administered to a subject. Optionally, the antigen or APC-chemotaxin is administered in a delivery vehicle such as a physiologically acceptable excipient.

### A. Administration and Dose Scheduling

As noted *supra,* in one embodiment of the invention an antigen is administered simultaneously with the chemotactic composition. In an alternative embodiment, the antigen and the chemotactic composition are administered at different times, typically to the same site. For example, the chemotactic composition (without the antigen) can be administered between about 15 m and about 96 h prior to the administration of the antigen, more often between about 15 m and about 48 h, more often between 24 h and 96 h, often between about 48 h and 72 h or between 72 h and 96 h prior to the administration of the antigen.

When the chemotactic composition and an antigen composition are injected at the same site in a subject, preferably the injections are within 2 cm of each other, preferably within 1 cm or within 0.5 cm of each other on the two dimensional surface of the body. In this embodiment, the administrations should also be done to a similar depth and to the same tissue layer (i.e. both injections should be subcutaneous or both intradermal). For intramuscular injections, the depth should be more precisely monitored to achieve a three dimensional equivalent placement of the APC-chemotaxin and the antigen to within 2 cm of each other, preferably to within 1 cm, and even better to within 0.5 cm. This is easily accomplished by physicians, nurses and other medically trained personal. The injection site can be marked with an indelible ink to assist the physician.

In one embodiment, only one dose (administration) of the composition is given. In another embodiment, the first administration is followed by boosting doses. Thus, in an embodiment, the APC-chemotaxin is administered in multiple doses, often in combination with administration of the antigen (e.g., by coadministration). Thus, in various embodiments, the APC-chemotaxin composition (optionally including antigen) is administered once, twice, three times, or more than three times. The number of doses administered to a subject is dependent upon the antigen, the extent of the disease, and the response of a subject to the chemotactic composition. In one embodiment of the invention, a second administration (booster) of the chemotactic composition and antigen occurs between about 7 days and 1 year after the original administration. In an embodiment, a second administration (booster) of the chemotactic composition and antigen occurs between about 14 days and 6 months after the original administration. Alternatively, a second administration (booster) of the chemotactic composition and antigen occurs between about 21 days and 3 months after the original administration, often between about 28 days and 2 months after the original administration. In one embodiment third administration (second booster) occurs between about 14 days and 10 years after the original administration, e.g., between about 14 days and 3 years after the original administration, often between about 21 days and 1 year after the original administration, very often between about 28 days and 6 months after the original administration. Subsequent boosters may be administered at 2 week intervals, or 1 month, 3 month or 6 month to 10 year intervals.

It will be recognized by those of ordinary skill that a variety of vaccine administration doses and schedules can be developed based upon the parameters discussed above and known in the art, and that determination of an effective amount and number of doses of chemotaxins of the invention, antigens, or some combination of chemotaxin(s) and antigen(s) for administration is well within the capabilities of those skilled in the art.

### B. Effective Dose

Typically, the an amount of APC-chemotaxin and antigen will be administered to the subject that is sufficient to immunize an animal against an antigen (i.e., an "immunologically effective dose" or a "therapeutically effective dose"). An amount adequate to accomplish an "immunologically effective dose" will depend on, e.g., the APC-chemotaxin and antigen composition, the manner of administration, the stage and severity of the disease being treated, the weight and general state of health of the patient, and the judgment of the prescribing physician.

The effective dose of antigen and APC-chemotaxin can be formulated in animal models to achieve an induction of an immune response using techniques that are well known in the art. One having ordinary skill in the art can readily optimize administration to humans based on animal data. When the APC-chemotaxin is a protein, such as a chemokine, a dose will typically be between about 1 fg and about 100 ug, often between about 1 pg and about 100 ug, more often between about 1 ng and about 50 ug, and usually between about 100 ng and about 50 ug. In some embodiments, the dose is between about 1 fg and about 100 ug per kg subject body weight, often between about 1 pg and about 100 ug, more often between about 1 ng and about 50 ug, and usually between about 100 ng and about 50 ug per kg subject body weight.

The amount of antigen that is administered will vary with the identity and characteristics of the antigen. In one embodiment, a chemotactic composition of the present invention contains one or more antigens and one or more chemotaxins at a molar or weight ratio of about 1:1000 or greater, chemotaxin to antigen. In another embodiment, the ratio of chemotaxin to antigen in the composition is between about 1:10 and 1:1000. In another embodiment, the ratio of antigen to chemotaxin in the composition is between about 1:10 and 1:1000, or greater than 1:1000 or greater. In another embodiment, the ratio of antigen to chemotaxin in the composition is between about 1:10 and 10:1.

### C. Carriers, Excipients, Conventional Adjuvants, Mode of Administration

The APC-chemotaxin-containing compositions of the invention may be administered in a variety of ways, as described herein. In various embodiments, the chemotactic composition includes carriers and excipients (i.e., pharmaceutically acceptable excipients) (including but not limited to buffers, carbohydrates, mannitol, proteins, polypeptides or amino acids such as glycine, antioxidants, bacteriostats, chelating agents, suspending agents, thickening agents and/or preservatives), other pharmaceutically acceptable auxiliary substances as required to approximate physiological conditions, such as buffering agents, tonicity adjusting agents, wetting agents and the like, and/or a conventional adjuvant (e.g., as discussed *supra*). It will be recognized that, while any suitable carrier known to those of ordinary skill in the art may be employed to administer the compositions of this invention, the type of carrier will vary depending on the mode of administration. Compounds may also be encapsulated within liposomes using well known technology. Biodegradable microspheres may also be employed as carriers for the pharmaceutical compositions of this invention. Suitable biodegradable microspheres are disclosed, for example, in U.S. Patent Nos. 4,897,268; 5,075,109; 5,928,647; 5,811,128; 5,820,883; 5,853,763; 5,814,344 and 5,942,252.

The compositions of the invention may be sterilized by conventional, well known sterilization techniques, or may be sterile filtered. The resulting aqueous solutions may be packaged for use as is, or lyophilized, the lyophilized preparation being combined with a sterile solution prior to administration.

The APC-chemotaxin composition of the invention may be administered in a variety of ways, including by injection (e.g., intradermal, subcutaneous, intramuscular, intraperitoneal and the like), by inhalation, by topical administration, by suppository, using a transdermal patch, orally.

When administration is by injection, the chemotaxin(s) may be formulated in aqueous solutions, preferably in physiologically compatible buffers such as Hanks's solution, Ringer's solution, or physiological saline buffer. The solution may contain formulatory agents such as suspending, stabilizing and/or dispersing agents. Alternatively, the chemotactic composition may be in powder form for constitution with a suitable vehicle, e.g., sterile pyrogen-free water, before use.

When administration is by inhalation, the chemotaxin(s) may be delivered in the form of an aerosol spray from pressurized packs or a nebulizer, with the use of a suitable propellant, e.g., dichlorodifluoromethane, trichlorofluoromethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the proteins and a suitable powder base such as lactose or starch.

When administration is by topical administration, the chemotactic composition may be formulated as solutions, gels, ointments, creams, suspensions, and the like, as are well-known in the art. In some embodiments, administration is by means of a transdermal patch.

When administration is by suppository (e.g, rectal or vaginal), compositions may also be formulated in compositions containing conventional suppository base.

When administration is oral, a composition can be readily formulated by combining the chemotaxin with pharmaceutically acceptable carriers well known in the art. A solid carrier, such as mannitol, lactose, magnesium stearate, and the like may be employed; such carriers enable the chemotaxin to be formulated as tablets, pills, dragees, capsules, liquids, gels, syrups, slurries, suspensions and the like, for oral ingestion by a subject to be treated. For oral solid formulations such as, for example, powders, capsules and tablets, suitable excipients include fillers such as sugars, cellulose preparation, granulating agents, and binding agents.

### 11. Vaccination for Monoclonal and Polyclonal Antibody Production

Methods of producing polyclonal and monoclonal antibodies, including binding fragments *(e.g.,* F(ab)₂) and single chain versions, are well known to those of skill in the art. *See, e.g.,* Coligan, 1991, CURRENT PROTOCOLS IN IMMUNOLOGY, Wiley/Greene, NY; and Harlow and Lane, 1989, ANTIBODIES: A LABORATORY MANUAL, Cold Spring Harbor Press, NY. However, many antigens are not capable of triggering an adequate antibody response in animals. In one embodiment, a composition comprising a chemotaxin of the invention and an antigen is administered to an animal as described herein, thus inducing or enhancing the immune response in the animal. Polyclonal or monoclonal antibodies are subsequently prepared by standard techniques.

### 12. Stimulation of Innate Immune Response

In another aspect of the invention, the compositions of the invention are administered to a subject to stimulate the innate immune response. The innate immune response is body's initial defense against pathogens and is elicited by a variety of cells including APCs (dendritic cells and the macrophages). These cells express surface and cytoplasmic receptors that recognize molecules of foreign origin (e.g., bacterial and viral nucleic acids, proteins, carbohydrates). Upon detecting these signals, the dendritic cells and macrophage elicit a defensive response that includes the release of cytokines (including interferons, TNFalpha, and IL12) and chemokines that attract cells such as immature dendritic cells, macrophage, NK cells, and granulocytes, to the site of challenge.

It is believed that the compositions of the invention are useful, not only to attract dendritic cells and other cells to the site of administration, but also that the compositions act to stimulate these cells into eliciting elements of the innate immune response to confer non-specific protection while the body is generating the adaptive response.

Thus, in one embodiment, a composition of the invention is administered prior or post exposure of an anticipated infection

### 13. Kits

In an aspect, the invention provides kits containing the following in a package or container: (1) an APC-chemotaxin composition of the invention; (2) a pharmaceutically acceptable adjuvant or excipient; (3) and antigen (e.g., a biologically pure antigen); (4) instructions for administration. Items (1) - (3) are typically in a container such as a vial, and may be frozen or lyophilized. Embodiments in which at least components (1) and (3) are found in the same container are also contemplated.

### 14. Selected Chemokine References

### mMIP-1α

Davatelis G, Tekamp-Olson P, Wolpe SD, Hennsen K, Luedke C, Gallegos C, Coit D, Merryweather J, Cerami A. Cloning and characterization of a cDNA for murine macrophage inflammatory protein (MIP), a novel monokine with inflammatory and chemokinetic properties. J Exp Med. 1988 Jun 1;167(6):1939-44.

### hMET-RANTES

Proudfoot AE, Power CA, Hoogewerf AJ, Montjovent MO, Borlat F, Offord RE, Wells TN. Extension of recombinant human RANTES by the retention of the initiating methionine produces a potent antagonist. J Biol Chem. 1996 Feb 2;271(5): 2599-603.

### mRANTES

Schall TJ, Simpson NJ, Mak JY Molecular cloning and expression of the murine RANTES cytokine: structural and functional conservation between mouse and man. Eur J Immunol. 1992 Jun;22(6):1477-81

### mMCP-5

Sarafi MN, Garcia-Zepeda EA, MacLean JA, Charo IF, Luster AD. Murine monocyte chemoattractant protein (MCP)-5: a novel CC chemokine that is a structural and functional homologue of human MCP-1. J Exp Med. 1997 Jan 6;185(1):99-109.

### mMARC

Thirion S, Nys G, Fiten P, Masure S, Van Damme J, Opdenakker G. Mouse macrophage derived monocyte chemotactic protein-3: cDNA cloning and identification as MARC/FIC. Biochem Biophys Res Commun. 1994 Jun 15;201(2):493-9

### mEotaxin

Rothenberg ME, Luster AD, Leder P. Murine eotaxin: an eosinophil chemoattractant inducible in endothelial cells and in interleukin 4-induced tumor suppression. Proc Natl Acad Sci U S A. 1995 Sep 12;92(19):8960-4.

### mMCP1(JE)

Van Damme J, Decock B, Bertini R, Conings R, Lenaerts JP, Put W, Opdenakker G, Mantovani A. Production and identification of natural monocyte chemotactic protein from virally infected murine fibroblasts. Relationship with the product of the mouse competence (JE) gene. Eur J Biochem. 1991 Jul 1;199(1):223-9.

### MTECK

Vicari AP, Figueroa DJ, Hedrick JA, Foster JS, Singh KP, Menon S, Copeland NG, Gilbert DJ, Jenkins NA, Bacon KB, Zlotnik A. TECK: a novel CC chemokine specifically expressed by thymic dendritic cells and potentially involved in T cell development. Immunity. 1997 Aug;7(2):291-301

### mMIP-2

Tekamp-Olson P, Gallegos C, Bauer D, McClain J, Sherry B, Fabre M, van Deventer S, Cerami A. Cloning and characterization of cDNAs for murine macrophage inflammatory protein 2 and its human homologues. J Exp Med. 1990 Sep 1;172(3):911-9.

### mBLC

Gunn MD, Ngo VN, Ansel KM, Ekland EH, Cyster JG, Williams LT. A B-cell-homing chemokine made in lymphoid follicles activates Burkitt's lymphoma receptor-1. Nature. 1998 Feb 19;391(6669):799-803.

### mMIP-1γ

Poltorak AN, Bazzoni F, Smirnova II, Alejos E, Thompson P, Luheshi G, Rothwell N, Beutler B J MIP-1 gamma: molecular cloning, expression, and biological activities of a novel CC chemokine that is constitutively secreted in vivo. Inflamm 1995;45(3):207-19

### mMIG

Farber JM, A macrophage mRNA selectively induced by gamma-interferon encodes a member of the platelet factor 4 family of cytokines, Proc Natl Acad Sci U S A 1990 Jul;87(14):5238-42.

### mMIP1-β

Sherry B, Tekamp-Olson P, Gallegos C, Bauer D, Davatelis G, Wolpe SD, Masiarz F, Coit D, Cerami A. Resolution of the two components of macrophage inflammatory protein 1, and cloning and characterization of one of those components, macrophage inflammatory protein 1 beta. J Exp Med. 1988 Dec 1;168(6):2251-9.

### vMIP-1

Nicholas J, Ruvolo VR, Bums WH, Sandford G, Wan X, Ciufo D, Hendrickson SB, Guo HG, Hayward GS, Reitz MS. Kaposi's sarcoma-associated human herpesvirus-8 encodes homologues of macrophage inflammatory protein-1 and interleukin-6. Nat Med. 1997 Mar;3(3):287-92

### mC10

Orlofsky, A, Berger, M.S., and Prystowsky, M.B. Novel expression pattern of a new member of the MIP-1 family of cytokine-like genes. Cell Regulation, Vol 2, p403-412, 1991.

### mMDC

Schaniel, C, E. Pardali, F. Sallusto, M. Speletas, C. Ruedl, T. Shimizu, T. Seidl, J. Andersson, F. Melchers, A. G. Rolink, and P. Sideras. Activated Murine B Lymphocytes and Dendritic Cells Produce a Novel CC Chemokine which Acts Selectively on Activated T Cells J. Exp. Med., Volume 188, Number 3, August 3, 1998 451-463

### hLeukotactin

Youn BS, Zhang SM, Lee EK, Park DH, Broxmeyer HE, Murphy PM, Locati M, Pease JE, Kim KK, Antol K, Kwon BS. Molecular cloning of leukotactin-1: a novel human beta-chemokine, a chemoattractant for neutrophils, monocytes, and lymphocytes, and a potent agonist at CC chemokine receptors 1 and 3. J Immunol. 1997 Dec 1;159(11):5201-5

### bMIP-1β

Lipes, MA. *et al*., (1988) Identification, cloning, and characterization of an immune activation gene. Proc. Natl. Acad. Sci. U.S.A 85:9704.

### hMCP-2

Van Damme J, Proost P, Lenaerts JP, Opdenakker G. Structural and functional identification of two human, tumor-derived monocyte chemotactic proteins (MCP-2 and MCP-3) belonging to the chemokine family. J Exp Med. 1992 Jul 1;176(1):59-65.

### hMCP-3

Van Damme J, Proost P, Lenaerts JP, Opdenakker G. Structural and functional identification of two human, tumor-derived monocyte chemotactic proteins (MCP-2 and MCP-3) belonging to the chemokine family. J Exp Med. 1992 Jul 1;176(1):59-65.

### hMIP-3α

Hieshima K, Imai T, Opdenakker G, Van Damme J, Kusuda J, Tei H, Sakaki Y, Takatsuki K, Miura R, Yoshie O, Nomiyama H. Molecular cloning of a novel human CC chemokine liver and activation-regulated chemokine (LARC) expressed in liver. Chemotactic activity for lymphocytes and gene localization on chromosome 2.

### hMIP-3β

Yoshida R, Imai T, Hieshima K, Kusuda J, Baba M, Kitaura M, Nishimura M, Kakizaki M, Nomiyama H, Yoshie O. Molecular cloning of a novel human CC chemokine EBI1-ligand chemokine that is a specific functional ligand for EBI1, CCR7. J Biol Chem. 1997 May 23;272(21):13803-9

### hRANTES

Schall, T*. et al*., (1988) A human T cell-specific molecule is a member of a new gene family. J. Immunol. 141:1018.

### hMIP1α

Obaru K, Fukuda M, Maeda S, Shimada K. A cDNA clone used to study mRNA inducible in human tonsillar lymphocytes by a tumor promoter. J Biochem (Tokyo). 1986 Mar;99(3):885-94.

### hMIP1α (70aa)

Irving et al., 1990, Two inflammatory mediator cytokine genes are closely linked and variably amplified on chromosome 17q. Nuc. Acid Res. 18:3261-70

### hHCC-1

Schulz-Knappe P, Magert HJ, Dewald B, Meyer M, Cetin Y, Kubbies M, Tomeczkowski J, Kirchhoff K, Raida M, Adermann K, et al. HCC-1, a novel chemokine from human plasma. J Exp Med. 1996 Jan 1;183(1):295-9.

### hMPIF-1

Forssmann U, Delgado MB, Uguccioni M, Loetscher P, Garotta G, Baggiolini M. CKbeta8, a novel CC chemokine that predominantly acts on monocytes. FEBS Lett. 1997 May 19;408(2):211-6

### hMPIF-1 (22-137)

Youn BS, Zhang SM, Broxmeyer HE, Cooper S, Antol K, Fraser M Jr, Kwon BS. Characterization of CKbeta8 and CKbeta8-1: two alternatively spliced forms of human beta-chemokine, chemoattractants for neutrophils, monocytes, and lymphocytes, and potent agonists at CC chemokine receptor 1. Blood. 1998 May 1;91(9):3118-26.

### hMPIF-1 (46-137)

Macphee CH, Appelbaum ER, Johanson K, Moores KE, Imburgia CS, Fornwald J, Berkhout T, Brawner M, Groot PH, O'Donnell K O'Shannessy D, Scott G, White JR. Identification of a truncated form of the CC chemokine CK beta-8 demonstrating greatly enhanced biological activity. J Immunol. 1998 Dec 1;161(11):6273-9.

### hMIP-1δ

Wang, W. *et al*., (1998) Molecular cloning and functional characterization of human MIP-1 delta, a new C-C chemokine related to mouse CCF-18 and C10. J. Clin. Immunol. 18(3):214.

### hMCP-4

Uguccioni, M. *et al*., (1996) Monocyte chemotactic protein 4 (MCP-4), a novel structural and functional analogue of MCP-3 and eotaxin. J. Exp. Med. 183:2379.

### m6Ckine

Hedrick JA, Zlotnik A. Identification and characterization of a novel beta chemokine containing six conserved cysteines. J Immunol. 1997 Aug 15; 159(4): 1589-93.

### hSDF1α

Tashiro K, Tada H, Heilker R, Shirozu M, Nakano T, Honjo T Signal sequence trap: a cloning strategy for secreted proteins and type I membrane proteins. Science. 1993 Jul 30;261(5121):600-3.

Nagasawa T, Kilcutani H, Kishimoto T. Molecular cloning and structure of a pre-B-cell growth-stimulating factor. Proc Natl Acad Sci U S A. 1994 Mar 15;91(6):2305-9.

### hSDF1β

Tashiro K, Tada H, Heilker R, Shirozu M, Nakano T, Honjo TSignal sequence trap: a cloning strategy for secreted proteins and type I membrane proteins. Science. 1993 Jul 30;261(5121):600-3.

### mSDF1α

Bleul CC, Fuhlbrigge RC, Casasnovas JM, Aiuti A, Springer TA. A highly efficacious lymphocyte chemoattractant, stromal cell-derived factor 1 (SDF-1). J Exp Med. 1996 Sep 1;184(3):1101-9.

### vMCK-2

Saederup N, Lin YC, Dairaghi DJ, Schall TJ, Mocarski ES. Cytomegalovirus-encoded beta chemokine promotes monocyte-associated viremia in the host. Proc Natl Acad Sci U S A. 1999 Sep 14;96(19):10881-6.

MacDonald MR, Burney MW, Resnick SB, Virgin HW. Spliced mRNA encoding the murine cytomegalovirus chemokine homolog predicts a beta chemokine of novel structure. J Virol. 1999 May;73(5):3682-91.

See also, GenBank, e.g., accession no. P10147 (hMIP1α), accession no. P10855 (mMIP-1α), accession no. P13501 (hRANTES), accession no. P30882 (mRANTES), accession no. Q16627 (hHCC-1), accession no. P55773 (hMPIF-1), accession no. Q16663 (hMIP-1δ), accession no. Q99616 (hMCP-4), accession no. Q32401 (mMCP-5), accession no. Q03366 (mMARC), accession no. P48298 (mEotaxin), accession no. P10148 (mMCP-1(JE)), accession no. 035903 (mTECK), accession no. P10889 (mMIP-2), accession no. AF044196 (mBLC), accession no. P18340 (mMIG), accession no. P14097 (mMIP-1β), accession no. P80075 (hMCP-2), accession no. P80098 (hMCP-3), accession no. P78556 (hMIP-3α), accession no. Q99731 (hMIP-3β), accession no. P27784 (mC10), accession no. AJ238238 (mMDC), accession no. P13236 (hMIP-1β), and accession no. P51670 (mMIP-1γ).

### 15. Examples

### Example 1

### Chemotaxis Assays

Chemotaxis assays are carried out using purified cells and a 96-well chemotaxis microchamber (ChemoTx®, NeuroProbes, Inc, Gaithersbrug MD). In this assay, a porous polycarbonate filter is used to allow formation of chemoattractant concentration gradient across the filter, as well as to allow cells to migrate into the filter or through into the lower well.

To perform an immature dendritic cell chemotaxis assay, 29 ul of a candidate or known APC-chemotaxin at 0, 1, 10 and 100 nM CHECK concentrations is placed in the wells of the lower chamber. The filter is placed on the top of the chamber, so that the chemoattractant solution touches the under side of the filter. Day 7 immature dendritic cells are harvested, washed once with chemotaxis buffer which contains 0.1% BSA (Sigma) in HBSS (Life Technologies), with Ca⁺⁺ and Mg⁺⁺), and finally resuspended in chemotaxis buffer at 5x10⁶ per ml. Twenty microliters of cells is carefully placed onto the filter. Migration is allowed to proceed for 90 minutes at 37°C in a tissue culture incubator. Migration is terminated by removing non-migrating cells on the top of the filter using a rubber scrapper. The filter is removed from the apparatus and rinsed with DPBS. (The lower chamber is inspected microscopically to determine if any cells have migrated into the wells. If significant number of cells are present in the wells, quantification is done in the wells as well as the filter.) To detect cells that have migrated into the polycarbonate filter in the immature dendritic cell migration assay, the filter, after being removed from the apparatus and rinsed with DPBS (Hyclone), and stained with a cell staining solution such as the Hema3 staining kit (Fisher Scientific). The filter is immersed into the three separate solutions sequentially, each for approximately 5 seconds. After the last solution, the filter is rinsed in water several times. The filter is allowed to air dry and the signal is determined by reading the filter on a plate reader (Molecular Devices) at wavelength 540 nm. The magnitude of migration is calculated as the ratio of absorbance between the wells with chemoattractants and the wells with chemotaxis buffer alone.

### Example 2

### Chemokine injection Into Mice

This example describes an *in vivo* assay in which the ability of several chemokines to attract dendritic cells is demonstrated.

The following chemokines were obtained from R&D Systems (Minneapolis, MN): MCP2, MCP3, MIP1β, MIP3α, MIP3β, Rantes, mMIG, mMDC, mC10, vMIP1. Each chemokine (2 ug in PBS) was injected intradermally into a different BALB/c mouse. One mouse received a control injection of PBS with no chemokine. 72 hours after injection, the mice were euthanized and the area around the injection site was excised and subjected to immunohistology. Frozen sections were stained with anti-DEC-205 antibody (available from Bio-Whittaker Molecular Applications, Rockland, ME; Kraal et al., 1986, J. Exp. Med. 163:981), which recognizes a surface molecule specific to dendritic cells. A relative staining number on a scale of 0 to 5 were assigned to each section (0 = lowest infiltration, 5 = highest infiltration). Results are shown in Table 4.

Several chemokines elicited a DEC-205+ (dendritic cell) infiltration to the site of injection after 72 hours when injected intradermally into the mouse. Of these MCP3, MIP3β, mMIG, mMDC, mC10, and viral MIP1 showed DC infiltration, with MCP3, mMDC, and mC10 showing the best infiltration.

**Table 4**

| Chemokine | DC Infiltration |
|---|---|
| MCP2 | 0 |
| MCP3 | 3 |
| MIP1b | 0 |
| MIP3a | 0 |
| MIP3b | 1 |
| Rantes | 0 |
| mMIG | 2 |
| mMDC | 3 |
| mC10 | 4 |
| vMIP1 | 0 |
| PBS | 0 |

### Example 3

### Chemokine Injection Into Rhesus monkeys

This example demonstrates that certain chemokines injected intradermally to a non-human primate elicit mononuclear infiltration to the site of injection.

Coded, sterile chemokines (2ug in PBS) were injected intradermally (100u1 injection volume) into the upper arm of a Rhesus macaque under anasthesia. In each case, two monkeys were each given two injections of the same chemokine to two different locations (e.g. left arm versus right arm). After 72 hours one injection site was punch biopsied and separated into an edge and a center sample and both samples were prepared for immunohistology. After 96 hours the other injection site was punch biopsied and separated into an edge and a center sample and both samples were prepared for immunohistology. Each row on Table 5 represents a single animal. A section of the prepared tissue was hematoxalin and eosin stained, and mononuclear cells identified based on cellular morphology (e.g. being mononuclear in contrast to polynuclear). GM-CSF (200ug doses) and Rantes (20ug doses) were use as positive controls. Injection of PBS and analysis of non-injected tissues were used as a negative control.

Mononuclear infiltration was score based on the following scale of 0 to 5: 0= a very mild perivascular mononuclear inflammatory infiltrate seen throughout the dermis; 1= a mild perivascular mononuclear inflammatory infiltrate seen throughout the dermis; 2= a mild/moderate perivascular mononuclear inflammatory infiltrate seen throughout the dermis; 3= a moderate perivascular mononuclear inflammatory infiltrate seen throughout the dermis; 4= an extensive perivascular mononuclear inflammatory infiltrate seen throughout the dermis; 5= a florid perivascular mononuclear inflammatory infiltrate seen throughout the dermis. intermediate scores are indicates, e.g., "2/3" represents a score between 2 and 3.

The results are shown in Table 5. GM-CSF (200ug dose) showed between a 2 and 3 level of infiltration at 72 hours. RANTES (20ug) dose showed between a 2 and 3 level of infiltration at 72 hours. Additional internally coded samples of RANTES (20ug dose) showed up to a 2 level of infiltration. Negative controls (PBS) usually had a 0 level of infiltrate

Of the chemokines tested, MCP-2, MCP-3, MIP-1β, MIP3α, MIP3β, Rantes, mMIG, mMDC, mC10, and viral MIP1 showed mononuclear infiltration, with mMDC, mC10, and viral MIP1 showing the highest level of infiltration. In general, greater infiltration was apparent after 72 hours than after 96 hours.

**Table 5**

| | 72 h | | 96 h | |
|---|---|---|---|---|
| Chemokine | Center | Edge | Center | Edge |
| | | | | |
| GM-CSF (200 ug)* | 0 | 0 | 3 | 2/3 |
| Rantes (20 ug)* | 0/1 | 1 | 2/3 | 1/2 |
| | | | | |
| MCP2 | 1/2 | 1 | 1/2 | 1 |
| MCP2 | 1 | 0/1 | 0 | 0 |
| | | | | |
| MCP3 | 1/2 | 0 | Not Determined | 0 |
| MCP3 | 0/1 | 0/1 | 0/1 | 1 |
| | | | | |
| MIP1b | 0 | 0/1focal | 1 | 0 |
| MIP1b | 1/2focal | 0/1 | 0 | 0 |
| | | | | |
| MIP3a | 1 | 1/2 | 0/1 | 0/1 |
| MIP3a | 2 | 1/2focal | 1/2 | 0/1 |
| | | | | |
| MIP3b | 2 | 1 | 0 | ½ |
| MIP3b | 1/2 | 1/2focal | - 2 | 1/2focal |
| | | | | |
| Rantes | 0 | 2 | 0 | 1/2focal |
| Rantes | 1/2 | 0/1 | 1/2focal | 0/1 |
| | | | | |
| mMIG | 0/1 | 1 | 0/1 | 0/1 |
| mMIG | 0 | 0/2focal | 0 | 0/1 |
| | | | | |
| mMDC | 1/2 | 1/2 | 0 | 1 |
| mMDC | 0/1 | 2/3focal | 2 | 2/3focal |
| | | | | |
| mC10 | 3/4 | 1/2 | 1/2focal | 1 |
| mC10 | 3/4 | 1/2 | 1/2 | 1/2focal |
| | | | | |
| vMIP1 | 2 | 0/1 | 0/1 | 1 |
| vMIP1 | 2 | 1/2 | 2/3 | 2/3 |

| | | | | |
|---|---|---|---|---|
| Key: "*" used as positive controls. Each horizontal row represents a separate Rhesus macaque and the histology scores of the 4 samples assayed form each animal. "Focal" one vessel in the section showed prominent perivascular inflammation (i.e., one section of the slide around a vessel showed prominent infiltration, where as the rest of the slide was more or less normal). "Center" and "Edge" refer to where the tissue section came from. "Center" tissue sections were taken at the site of the intradermal injection; "Edge" tissue sections were taken further from the injection site, towards the edge of the wheal generated by injection. | | | | |

### Example 4

### Chemokine Injection into Rhesus monkeys

In a second experiment, different amounts (approximately 8, 2.4, or 0.8 ug in 100ul PBS) of different chemokines were injected intradermally in Rhesus macaques under anesthesia. 24 or 48 hours later, 6 mm skin punch biopsies were taken using aseptic technique, then bisected and prepared for analysis. One portion of the biopsy was embedded in OCT compound, flash frozen and stored at -70°C. The other portion of the biopsy was fixed in formalin and embedded in paraffin wax; subsequently, sections were stained with hematoxylin and eosin and microscopically examined for cell infiltration into the dermis (Table 6). As a negative control, monkeys were injected with PBS lacking chemokines.

Mononuclear cell infiltration in the dermis was scored based on a scale of 0 to 4, as follows: 0, no infiltration; 1, slight infiltration; 2, moderate infiltration; 3, substantial infiltration; 4, severe infiltration. As indicated in Table 6, chemokines mC10 and vMCK-2 caused substantial mononuclear cell infiltration, especially at the 24 hour time-point. It was notable that a chemokine encoded by a mouse virus (vMCK-2) exhibited potent activity in primate cells. Infiltrating cells were observed predominantly in the adipose tissue, although cells were also noted in the subcutaneous region and, in the case of vMCK-2 at 48 hours, in the collagen matrix of the superficial dermis. Chemokines mMDC and vMIP-1 caused less inflammation than mC10 and vMCK-2.

**Table 6**

| Chemokine | 24 hr | 48 hr |
|---|---|---|
| | | |
| mC10:0.8ug | 2 | 0 |
| 2.4ug | 1 | 2 |
| 8ug | 3 | 2 |
| | | |
| mMDC: 0.8ug | 2 | 0 |
| 2.4ug | 0 | 0 |
| 8ug | 2 | 0 |
| | | |
| vMIP-1: 0.8ug | 0 | 0 |
| 2.4ug | 1 | 0 |
| 8ug | 0 | 0 |
| | | |
| vMCK-2:0.8ug | 2 | 2 |
| 2.4ug | 2 | 2 |
| 8ug | 4 | 3 |

### Example 5

### Design of Hvbrikines

"Hybrikines" are chimeric chemokine polypeptides designed to generate novel molecules with the appropriate, desired, and enhanced qualities. Usually, the amino acid sequence of a desired hybrikines is determined, and the molecule is produced by chemical synthesis, In this example, the sequences of chemokines hMCP-2, mC10 and mMDC were conceptually divided into functional domains based on the spacing of the invariant cysteine residues. Chimeric sequences were prepared by swapping domains between these molecules. The amino termini region (all amino acids of the mature native protein amino terminal of the first conserved cysteine residue) were swapped onto the remaining portion of a different chemokine molecule to create hybrids. As shown in Fig. 2, the amino terminal regions between mC10 and hMCP-2 to create two novel molecules: the mC10/hMCP2 and hMCP2/mC10 "hybridkine" molecules. In addition, the amino terminal regions between mMDC and hMCP-2 are swapped to create an additional two novel molecules: the mMDC/hMCP2 and hMCP2/mMDC "hybrikine" molecules. These polypeptides were synthesized chemically using classical Fmoc peptide chemistry. The polypeptides are used in *in vitro* and *in vivo* chemotaxis assays to determine their chemotactic properties.

The present invention provides novel methods and materials relating to APC-chemotactic compositions and therapeutic and prophylactic immunization. While specific examples have been provided, the above description is illustrative and not restrictive. Many variations of the invention will become apparent to those of skill in the art upon review of this specification.

## Claims

1. The use of an antigen-presenting cell chemotaxin (APC-chemotaxin) and an antigen in the manufacture of a medicament, wherein the APC-chemotaxin is:
(a) a chemokine selected from murine cytomegalovirus chemokine-2 (vMCK-2) and mC10;
(b) a variant of (a) that does not change the chemotactic properties of the polypeptide; or
(c) a polynucleotide encoding (a) or (b).

2. The use of claim 1, wherein the composition further comprises an additional chemokine.

3. The use of claim 1, wherein the composition comprises both vMCK-2 and mC10.

4. The use of claim 1, wherein the APC-chemotaxin is encoded by a polynucleotide produced by in vitro recombination of polynucleotides that encode vMCK-2 or mC10 and another naturally occurring chemokine.

5. The use of claim 1, wherein the antigen is derived from a microbial pathogen or is a tumor-associated antigen.

6. The use of claim 5, wherein the microbial pathogen is a bacterium or a virus.

7. The use of claim 1, wherein the APC-chemotaxin is vMCK-2.

8. The use of claim 1, wherein the APC-chemotaxin is mC10.

9. The use of claim 1, wherein the APC-chemotaxin is a polynucleotide encoding vMCK-2.

10. The use of claim 1, wherein the APC-chemotaxin is a polynucleotide encoding mC10.

11. A vaccine composition comprising a substantially purified chemokine polypeptide and an antigenic polypeptide, wherein the chemokine polypeptide is murine cytomegalovirus chemokine-2 (vMCK-2) or a variant thereof that does not change the chemotactic properties of the polypeptide, or mC10 or a variant thereof that does not change the chemotactic properties of the polypeptide.

12. The vaccine composition of claim 11, wherein the chemokine polypeptide is vMCK-2.

13. The vaccine composition of claim 11, wherein the chemokine polypeptide is mC10.

14. A composition comprising a substantially purified antigen-presenting cell chemotaxin (APC-chemotaxin) and an antigen, wherein said APC-chemotaxin is:
(a) a chemokine selected from the group consisting of murine cytomegalovirus chemoldne-2 (vMCK-2) or mC10;
(b) a variant of (a) that does not change the chemotactic properties of the polypeptide; or
(c) a polynucleotide encoding (a) or (b).

15. The composition of claim 14, further comprising a pharmaceutically acceptable excipient or a conventional adjuvant

16. The composition of claim 14, further comprising an additional chemokine.

17. The composition of claim 14 that comprises both vMCK-2 and mC10.

18. The composition of claim 14, wherein the antigen is derived from a microbial pathogen or is a tumor-associated antigen.

19. The composition of claim 18, wherein the microbial pathogen is a bacterium or a virus.

20. The composition of claim 14, wherein the APC-chemotaxin is vMCK-2.

21. The composition of claim 14, wherein the APC-chemotaxin is mC10.

22. The composition of claim 14, wherein the APC-chemotaxin is a polynucleotide encoding vMCK-2.

23. The composition of claim 14, wherein the APC-chemotaxin is a polynucleotide encoding mC10.

24. A method of formulating a composition capable of inducing an immune response to a specified antigen in a subject, comprising combining an antigen-presenting cell chemotaxin (APC-chemotaxin), the specified antigen and a pharmaceutically acceptable excipient or a conventional adjuvant, wherein the APC-chemotaxin is:
(a) a chemokine selected from murine cytomegalovirus chemokine-2 (vMCK-2) and mC10;
(b) a variant of (a) that does not change the chemotactic properties of the polypeptide; or
(c) a polynucleotide encoding (a) or (b).

25. The method of claim 24, wherein the specified antigen is derived from a microbial pathogen or is a tumor-associated antigen.

26. The method of claim 25, wherein the microbial pathogen is a bacterium or a virus.

27. The method of claim 24, wherein the APC-chemotaxin is vMCK-2.

28. The method of claim 24, wherein the APC-chemotaxin is mC10.

29. The method of claim 24, wherein the APC-chemotaxin is a polynucleotide encoding vMCK-2.

30. The method of claim 24, wherein the APC-chemotaxin is a polynucleotide encoding mC10.

## Patentansprüche

1. Verwendung eines Antigen-präsentierenden Zell-Chemotaxins (APC-Chemotaxin) und eines Antigens bei der Herstellung eines Medikaments, wobei das APC-Chemotaxin ist:
(a) ein Chemokin, ausgewählt aus Maus-Cytomegalovirus Chemokin-2 (vMCK-2) und mC10;
(b) eine Abart von (a), die die chemotaktischen Eigenschaften des Polypeptids nicht ändert; oder
(c) ein (a) oder (b) kodierendes Polynucleotid.

2. Verwendung nach Anspruch 1, worin die Zusammensetzung ferner ein zusätzliches Chemokin enthält.

3. Verwendung nach Anspruch 1, worin die Zusammensetzung sowohl vMCK-2 als auch mC10 enthält.

4. Verwendung nach Anspruch 1, worin das APC-Chemotaxin durch ein Polynucleotid kodiert ist, das durch in vitro-Rekombination von Polynucleotiden, die vMCK-2 oder mC10 kodieren und einem anderen natürlich vorkommenden Chemokin erzeugt wird.

5. Verwendung nach Anspruch 1, worin das Antigen von einem mikrobiellen Erreger stammt oder ein Tumor-assoziiertes Antigen ist.

6. Verwendung nach Anspruch 5, worin der mikrobielle Erreger ein Bakterium oder ein Virus ist.

7. Verwendung nach Anspruch 1, worin das APC-Chemotaxin vMCK-2 ist.

8. Verwendung nach Anspruch 1, worin das APC-Chemotaxin mC10 ist.

9. Verwendung nach Anspruch 1, worin das APC-Chemotaxin ein vMCK-2 kodierendes Polynucleotid ist.

10. Verwendung nach Anspruch 1, worin das APC-Chemotaxin ein mC10 kodierendes Polynucleotid ist.

11. Impfstoffzusammensetzung, umfassend ein im Wesentlichen gereinigtes Chemokin-Polypeptid und ein antigenes Polypeptid, worin das Chemokin-Polypeptid Maus-Cytomegalovirus Chemokin-2 (vMCK-2) oder eine Abart davon ist, die die chemotaktischen Eigenschaften des Polypeptids nicht ändert, oder mC10 oder eine Abart davon, die die chemotaktischen Eigenschaften des Polypeptids nicht ändert.

12. Impfstoffzusammensetzung nach Anspruch 11, worin das Chemokin-Polypeptid vMCK-2 ist.

13. Impfstoffzusammensetzung nach Anspruch 11, worin das Chemokin-Polypeptid mC10 ist.

14. Zusammensetzung, umfassend ein im Wesentlichen gereinigtes Antigen-präsentierendes Zell-Chemotaxin (APC-Chemotaxin) und ein Antigen, worin das APC-Chemotaxin ist:
(a) ein Chemokin, ausgewählt aus der Gruppe bestehend aus Maus-Cytomegalovirus Chemokin-2 (vMCK-2) und mC10;
(b) eine Abart von (a), die die chemotaktischen Eigenschaften des Polypeptids nicht ändert; oder
(c) ein (a) oder (b) kodierendes Polynucleotid.

15. Zusammensetzung nach Anspruch 14, weiterhin umfassend einen pharmazeutisch akzeptablen Hilfsstoff oder einen üblichen Zusatz.

16. Zusammensetzung nach Anspruch 14, weiterhin umfassend ein zusätzliches Chemokin.

17. Zusammensetzung nach Anspruch 14, die sowohl vMCK-2 als auch mC10 enthält.

18. Zusammensetzung nach Anspruch 14, worin das Antigen von einem mikrobiellen Erreger stammt oder ein Tumor-assoziiertes Antigen ist.

19. Zusammensetzung nach Anspruch 18, worin der mikrobielle Erreger ein Bakterium oder ein Virus ist.

20. Zusammensetzung nach Anspruch 14, worin das APC-Chemotaxin vMCK-2 ist.

21. Zusammensetzung nach Anspruch 14, worin das APC-Chemotaxin mC10 ist.

22. Zusammensetzung nach Anspruch 14, worin das APC-Chemotaxin ein vMCK-2 kodierendes Polynucleotid ist.

23. Zusammensetzung nach Anspruch 14, worin das APC-Chemotaxin ein mC10 kodierendes Polynucleotid ist.

24. Verfahren zur Formulierung einer Zusammensetzung, die in der Lage ist, eine Immunantwort in einem vorgegebenen Antigen bei einer Testperson zu induzieren, umfassend das Kombinieren eines Antigen-präsentierenden Zell-Chemotaxins (APC-Chemotaxin), des vorgegebenen Antigens und eines pharmazeutisch akzeptablen Hilfsstoff oder eines üblichen Zusatzes, worin das APC-Chemotaxin ist:
(a) ein Chemokin, ausgewählt aus Maus-Cytomegalovirus Chemokin-2 (vMCK-2) und mC10;
(b) eine Abart von (a), die die chemotaktischen Eigenschaften des Polypeptids nicht ändert; oder
(c) ein (a) oder (b) kodierendes Polynucleotid.

25. Verfahren nach Anspruch 24, worin das vorgegebene Antigen von einem mikrobiellen Erreger stammt oder ein Tumor-assoziiertes Antigen ist.

26. Verfahren nach Anspruch 25, worin der mikrobielle Erreger ein Bakterium oder ein Virus ist.

27. Verfahren nach Anspruch 24, worin das APC-Chemotaxin vMCK-2 ist.

28. Verfahren nach Anspruch 24, worin das APC-Chemotaxin mC10 ist.

29. Verfahren nach Anspruch 24, worin das APC-Chemotaxin ein vMCK-2 kodierendes Polynucleotid ist.

30. Verfahren nach Anspruch 24, worin das APC-Chemotaxin ein mC10 kodierendes Polynucleotid ist.

## Revendications

1. Utilisation d'une cellule présentatrice d'antigène-chimiotaxine (APC-chimiotaxine) et d'un antigène pour la fabrication d'un médicament, dans laquelle l'APC-chimiotaxine est :
(a) une chimiokine sélectionnée parmi la chimiokine-2 du cytomégalovirus murin (vMCK-2) et mC10,
(b) un variant de (a) qui ne change pas les propriétés chimiotactiques du polypeptide, ou
(c) un polynucléotide codant pour (a) ou (b).

2. Utilisation selon la revendication 1, dans laquelle la composition comporte de plus une chimiokine supplémentaire.

3. Utilisation selon la revendication 1, dans laquelle la composition comporte à la fois vMCK-2 et mC10.

4. Utilisation selon la revendication 1, dans laquelle l'APC-chimiotaxine est codée par un polynucléotide produit par une recombinaison in vitro de polynucléotides qui codent pour vMCK-2 ou mC10 et une autre chimiokine apparaissant naturellement.

5. Utilisation selon la revendication 1, dans laquelle l'antigène est dérivé d'un pathogène microbien ou est un antigène associé à une tumeur.

6. Utilisation selon la revendication 5, dans laquelle le pathogène microbien est une bactérie ou un virus.

7. Utilisation selon la revendication 1, dans laquelle l'APC-chimiotaxine est vMCK-2.

8. Utilisation selon la revendication 1, dans laquelle l'APC-chimiotaxine est mC10.

9. Utilisation selon la revendication 1, dans laquelle l'APC-chimiotaxine est un polynucléotide codant pour vMCK-2.

10. Utilisation selon la revendication 1, dans laquelle l'APC-chimiotaxine est un polynucléotide codant pour mC10.

11. Composition vaccinale comportant un polypeptide de chimiokine essentiellement purifié et un polypeptide antigénique, dans laquelle le polypeptide de chimiokine est la chimiokine-2 du cytomégalovirus murin (vMCK-2) ou un variant de celle-ci qui ne change pas les propriétés chimiotactiques du polypeptide, ou mC10 ou un variant de celui-ci qui ne change pas les propriétés chimiotactiques du polypeptide.

12. Composition vaccinale selon la revendication 11, dans laquelle le polypeptide de chimiokine est vMCK-2.

13. Composition vaccinale selon la revendication 11, dans laquelle le polypeptide de chimiokine est mC10.

14. Composition comportant une cellule présentatrice d'antigène-chimiotaxine (APC-chimiotaxine) essentiellement purifiée et un antigène, dans laquelle ladite APC-chimiotaxine est :
(a) une chimiokine sélectionnée parmi le groupe constitué de la chimiokine-2 du cytomégalovirus murin (vMCK-2) ou mC10,
(b) un variant de (a) qui ne change pas les propriétés chimiotactiques du polypeptide, ou
(c) un polynucléotide codant pour (a) ou (b).

15. Composition selon la revendication 14, comportant de plus un excipient pharmaceutiquement acceptable ou un adjuvant classique.

16. Composition selon la revendication 14, comportant de plus une chimiokine supplémentaire.

17. Composition selon la revendication 14, qui comporte à la fois vMCK-2 et mC10.

18. Composition selon la revendication 14, dans laquelle l'antigène est dérivé d'un pathogène microbien ou est un antigène associé à une tumeur.

19. Composition selon la revendication 18, dans laquelle le pathogène microbien est une bactérie ou un virus.

20. Composition selon la revendication 14, dans laquelle l'APC-chimiotaxine est vMCK-2.

21. Composition selon la revendication 14, dans laquelle l'APC-chimiotaxine est mC10.

22. Composition selon la revendication 14, dans laquelle l'APC-chimiotaxine est un polynucléotide codant pour vMCK-2.

23. Composition selon la revendication 14, dans laquelle l'APC-chimiotaxine est un polynucléotide codant pour mC10.

24. Procédé pour formuler une composition capable d'induire une réponse immunitaire à un antigène spécifié chez un sujet, comportant la combinaison d'une cellule présentatrice d'antigène-chimiotaxine (APC-chimiotaxine), l'antigène spécifié et un excipient pharmaceutiquement acceptable ou un adjuvant classique, dans lequel l'APC-chimiotaxine est :
(a) une chimiokine sélectionnée parmi la chimiokine-2 du cytomégalovirus murin (vMCK-2) et mC10,
(b) un variant de (a) qui ne change pas les propriétés chimiotactiques du polypeptide, ou
(c) un polynucléotide codant pour (a) ou (b).

25. Procédé selon la revendication 24, dans lequel l'antigène spécifié est dérivé d'un pathogène microbien ou est un antigène associé à une tumeur.

26. Procédé selon la revendication 25, dans lequel le pathogène microbien est une bactérie ou un virus.

27. Procédé selon la revendication 24, dans lequel l'APC-chimiotaxine est vMCK-2.

28. Procédé selon la revendication 24, dans lequel l'APC-chimiotaxine est mC10.

29. Procédé selon la revendication 24, dans lequel l'APC-chimiotaxine est un polynucléotide codant pour vMCK-2.

30. Procédé selon la revendication 24, dans lequel l'APC-chimiotaxine est un polynucléotide codant pour mC10.
